(19) Europäisches Patentamt
European Patent Office
Office européen des brevets

(11) EP 1 243 651 B1

(12) EUROPEAN PATENT SPECIFICATION

(45) Date of publication and mention of the grant of the patent:
**20.02.2008 Bulletin 2008/08**

(21) Application number: **00985852.3**

(22) Date of filing: **25.12.2000**

(51) Int Cl.:
***C12N 15/11*** (2006.01) ***C12Q 1/68*** (2006.01)
***A01K 61/00*** (2006.01) ***C07K 14/46*** (2006.01)
***A01K 67/00*** (2006.01) ***C12N 15/09*** (2006.01)
***C12N 15/12*** (2006.01) ***C12N 15/85*** (2006.01)
***G01N 33/00*** (2006.01)

(86) International application number:
**PCT/JP2000/009170**

(87) International publication number:
**WO 2001/048191 (05.07.2001 Gazette 2001/27)**

(54) **METHOD OF DETECTING SEXUAL DIFFERENTIATION DISRUPTOR**

VERFAHREN ZUR ERKENNUNG EINES AGENS, WELCHES DIE SEXUELLE ENTWICKLUNG UNTERBRICHT

TECHNIQUE DE DETECTION D'UN AGENT PERTURBATEUR DE DIFFERENTIATION SEXUELLE

(84) Designated Contracting States:
**AT BE CH CY DE DK ES FI FR GB GR IE IT LI LU MC NL PT SE TR**

(30) Priority: **28.12.1999 JP 37260299**

(43) Date of publication of application:
**25.09.2002 Bulletin 2002/39**

(73) Proprietor: **TAKARA BIO INC.**
**Otsu-shi,**
**Shiga 520-2193 (JP)**

(72) Inventors:
- **KANAMORI, Akira**
  **Nagoya-shi,**
  **Aichi 464-0816 (JP)**
- **KINOSHITA, Masato**
  **Kyoto-shi, Kyoto 606-0031 (JP)**
- **TAKASHIMA, Ryokichi**
  **Otsu-shi, Shiga 520-2141 (JP)**
- **CHONO, Hideto**
  **Moriyama-shi, Shiga 524-0036 (JP)**
- **MIZUTANI, Shigetoshi**
  **Gamo-gun, Shiga 521-1322 (JP)**
- **KONDO, Akihiro,**
  **101, Hamoparesu-Kusatsu**
  **Kusatsu-shi,**
  **Shiga 525-0025 (JP)**
- **KATO, Ikunoshin**
  **Uji-shi, Kyoto 611-0028 (JP)**

(74) Representative: **Schnappauf, Georg et al**
**Dr. Volker Vossius**
**Patent- und Rechtsanwaltskanzlei**
**Geibelstrasse 6**
**81679 München (DE)**

(56) References cited:
- **KANAMORI A: "Systematic identification of genes expressed during early oogenesis in medaka." MOLECULAR REPRODUCTION AND DEVELOPMENT. JAN 2000, vol. 55, no. 1, January 2000 (2000-01), pages 31-36, XP008032480 ISSN: 1040-452X -& KANAMORI A: "Systematic identification of genes expressed during early oogenesis in medaka." MOLECULAR REPRODUCTION AND DEVELOPMENT, [Online] 6 December 1999 (1999-12-06), XP002287390 Retrieved from the Internet: URL:http: //www3.interscience.wiley.com/cgi -bin/abstract/ 68000920/ABSTRACT> [retrieved on 2004-07-07]**
- **DATABASE EMBL [Online] 18 June 1999 (1999-06-18), KANAMORI A: "Oryzias latipes FIGalpha mRNA, complete cds." XP002287020 Database accession no. AF128805**
- **DATABASE EMBL [Online] 18 June 1999 (1999-06-18), KANAMORI A: "Oryzias latipes ZPC domain containing protein 1 mRNA, complete cds." XP002305973 Database accession no. AF128809**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **GRAY M A ET AL: "INDUCTION OF TESTIS-OVA IN JAPANESE MEDAKA (ORYZIAS LATIPES) EXPOSED TO P-NONYLPHENOL" ENVIRONMENTAL TOXICOLOGY AND CHEMISTRY, PERGAMON PRESS, US, vol. 16, no. 5, 1997, pages 1082-1086, XP002939716 ISSN: 0730-7268**
- **MURATA K ET AL: "Cloning of cDNA and estrogen-induced hepatic gene expression for choriogenin H, a precursor protein of the fish egg envelope (chorion)" PROCEEDINGS OF THE NATIONAL ACADEMY OF SCIENCES OF THE UNITED STATES OF AMERICA 1997 UNITED STATES, vol. 94, no. 5, 1997, pages 2050-2055, XP002287016 ISSN: 0027-8424**
- **BAROILLER J -F ET AL: "Endocrine and environmental aspects of sex differentiation in fish" CMLS CELLULAR AND MOLECULAR LIFE SCIENCES, vol. 55, no. 6-7, June 1999 (1999-06), pages 910-931, XP002287018 ISSN: 1420-682X**
- **ARCAND-HOY LISA D ET AL: "Fish reproduction: An ecologically relevant indicator of endocrine disruption" ENVIRONMENTAL TOXICOLOGY AND CHEMISTRY, vol. 17, no. 1, January 1998 (1998-01), pages 49-57, XP008032487 ISSN: 0730-7268**
- **SCHOLZ S ET AL: "17-alpha-ethinylestradiol affects reproduction, sexual differentiation and aromatase gene expression of the medaka (Oryzias latipes)" AQUATIC TOXICOLOGY (AMSTERDAM), vol. 50, no. 4, October 2000 (2000-10), pages 363-373, XP002287017 ISSN: 0166-445X**
- **MICHELLE A. GRAY ET AL.: 'Induction of testis-ova in japanese medaka (oryzias latipes) exposed to p-nonyphenol' ENVIRONMENTAL TOXICOLOGY AND CHEMISTRY vol. 16, no. 5, 1997, pages 1082 - 1086, XP002939716**
- **TSUTOMU SHIODA ET AL.: 'Naibunpitsu kakuran kagaku busshitsu screening hou to shite no himedaka wo mochiita tanki hanshoku shiken' NIPPON NAIBUNPITSU KAKURAN KAGAKU BUSSHISU GAKKAI vol. 2, 1999, page 66, XP002944157**
- **HIRONORI WADA ET AL.: 'Sex-linked inheritance of the if locus in the medaka fish (oryzias latipes) ' ZOOLOGICAL SCIENCE vol. 15, no. 1, 1998, pages 123 - 126, XP002939717**

SEQUENCING LISTING

<110> Takara Shuzo Co., Ltd.

<120> Method of detecting sexual differentiation disruptor

<130> 38-49

<140> EP 00 985 852.3
>141> 2000-12-25

<150> JP 11-372602
<151> 1999-12-28

<160> 27

<210> 1
<211> 1099
<212> DNA
<213> Oryzias latipes

<220>
<223> cDNA for gene 1

<400> 1

```
gtaaacggac agctagagtt cagctgaaaa gaagtgtaat tctattcgga cgtagtcttt 60
aaaaaaaaaa tgaaggtgcc agaggcggaa ttaatgagcg acattctgaa gaggctgacg 120
ggagagtctg ctctgccgct gtactgctgc atcgagaagt acaagcgcga gaggaacggc 180
ctctactttg tcgccgagga tttcactgaa accgtcaaaa aaagagaaat ggtcaacgcc 240
```

aaggaaagac tgagaataag gaacttgaac acaatgttct cccgactgaa gcgcatgctg 300

cctctaatgc aaccagacaa aaagccgagt aaagttgata cactcaaagc agccactgaa 360

tacattcgac ttcttcttgc tgttttgcgg gacactgaaa ataacaacac tgggacggat 420

tttctaaaga atgcaatcac ttatggtcag caggatggct tcgccaatga cctctggaga 480

atggacgatt tcttgaacct gtcagatgat cacatggagg atgggttcac catgccagca 540

gaacctgcag cagaggatgg agacatgact agactggtgt tgcagcattg tgtgatgcct 600

gcataccagt tcatcatcca agtagcgccc gatcaagctt cgagggatta attagccacc 660

gcctcccgac tgcacatccc aaccactgac ccgatgtcct tgctatcttg gacattgatg 720

acttgcactc tttttccttg acacttatta taaaatggct tgatttaaaa cctaaccctt 780

aatttttttt tcattattta gttgtacata cattggaagt tgatgcatct agtaaacata 840

cctaaaaaga actgctgctc taagagttct cattttgctt tcagctgtac tctatttgag 900

gaaatgactt aatttatgta tttttcttgt aattgtaatc ataaagcccc aatgaaaaag 960

atgcaaattt gtgacaattt gttgaggtca atgtgatcta agttgtcaat atgaattatc 1020

tgtttgaaaa cttgaatcta atttaaattc agaaatgtct aaatgtgttt agttaatgat 1080

caaataaaca agctttaag 1099

<210> 2
<211> 938
<212> DNA
<213> Oryzias latipes

<220>
<223> cDNA for gene 2

<400> 2

gaccgccgta gtggtgtcca cttcgattcc tggaaacctc gaggaaaaaa gtgaaataag 60

caacggaaag atcgggaatc ctgaggcttg catcaaacat cccttacaga acaggtggac 120

tctttggttt ttcaagaatg acaaaagcaa aacctggcag gccaaccttc gtctcatctc 180

tacgtttgac acagttgagg atttctgggc gttatacaat catattcaac tgtcaagtaa 240

cttgatgtct ggctgtgact actctctgtt taaggatggg attgaaccca tgtgggagga 300
tgaaaggaat cggcgtgggg gccgctggct catcaccctg tccaaacatc aacggaaaat 360
ggatctggac cggttctggt tggagacgct tttgtgtctc gtgggcgaag cctttgatga 420
tcacagcgat gatgtttgtg gagccgtcat caacatccga gccaaaggag ataaaatagc 480
aatatggacc acaaactgtg aaaacaaaga ggccatcaca cagattggtc gagtttataa 540
ggagaggtta ggcgttccac ccaaagtgat catcgggtac cagtcccacg cagacacggc 600
tacaaagagc agctccacaa ccaagaacaa gtttgttgct taaaggtttt gggtttgtct 660
tctttccttg ttttgttttt ttcaaagtaa ctgaaagttt attcaccaaa agccttttct 720
aaaatggaac aagtcttgcc tttactttgt cagaacattt gtttgtggtg agaatacaat 780
aaaaaaatag taggtgtaaa atcctaatga ttgacttcat catgggatag aaatgtaagc 840
agtttattgg aggtgattca tttgtgttcc aacaattctc tcagttttta ccttttttaag 900
ttgtttatgg agaattgctt aataaaggta catgaatt 938

<210> 3
<211> 2862
<212> DNA
<213> Oryzias latipes

<220>
<223> cDNA for gene 3

<400> 3

gctgcttcat cttcttggcc ttggtgtaaa agtcaaagcc aagttctggt ggttgagttc 60
tttccagttc ctttttccaa tgccttcaca ctgcaacatg cggaagcctg agaaaatgtt 120
tttgttattg aacctgatgg ctgcagtttc tgtcttgagt caagcatggc caaatgtgaa 180
gcatggtctg cagtcaacca atggagttag atcggactgt gcaggtaatc tgatgaggct 240
ttcattggat gaggctcttg cagttgggaa tcaacttcaa gtggaagcaa tcaatggcac 300
acaacgtatt ttggtgacac ccagtctggc tgcccagtgt ggatacagca tggagtctga 360
cccatgggga aacaccagga tctacatgtc tctgttgggt tgctttatgg ttagcaagga 420

tgagtccacc ttcagcacca gcttgaaact gcacatgtac aagcacagtc cctctgatgt 480
ggtcagccat gatgtgactc agacctgcag ctattctcgc tgggccttca gagatgtcct 540
ctgtgacagg aactacatgg aagtgtcagc tcacatcgct cccagtcaac aaacaaaagg 600
acaagttcag aacaaggaca actctcaaac aaataagctt cctggtgact ccaatgacgc 660
ccctggaatc tggaagatga ccttttacac tcctgaacct gttgcgatgg tcctgaaaga 720
agccgaacaa gctggttatg gtgcaacaat gagacaaact cgcctggcaa tcagaagccc 780
ctatcatact tcagagactt attctgaaga tgttgctgga gtccccatgg aaatcctcaa 840
agtgagcgtt taccacaaaa ctcaagaggg actgaatgtt gtcaacttgg cagctgcttg 900
ccccacaggt ggcattctct tcactgacga gttcatctcc tggcacatcc ctcgccgcgt 960
aactcctcta actgagggca aggtcaagat tgtagagctg tacatgggaa tcaatggaca 1020
gaggctggaa aaggctcaaa tagctgcaag aggttactcc ctctcaacca cagagttcca 1080
cattgttgtt gacatcccag tgggatcacc tgacggctac tacaagagcc atgctccaga 1140
cttcctgtac cacatcacct acaccattga gccaatgctt gaagttctat ggaggccaga 1200
gaatccccaa gaagaaacaa aatacaaaat tctgtttccc atcacaactc caccaatcct 1260
tcatccaatc aacacctttg accgcacaat cccagaagag agggtgttta atgtccatgt 1320
gggggctttc cttcatgatg tggtgctgaa gaacatcaca ttttctactg gagtccttac 1380
tgttgaagag tgcaatgcca aagggtttgc agtccaggaa catcttctct ccaacggcac 1440
aaaagtgttc tctattcaag tggcctttga caccgatccc atcctgaagc ataatcctga 1500
gcccttggtt acaacctact tcctccctct gatctttggt tttgctgttc tgcctgagga 1560
tctccctttt gcccacaaag tagagttgca ggcatctctg caggatgttg ttctgccaac 1620
actcactgga acctgtgacc aggagcactt cttcgtttcc gtcaagtacg gcagtcaagg 1680
gcatcacttc aagaccatgg tcggccatca ggacctgaca cctgaactgg gagagacact 1740
agcctatcag gacaatggca cacacttcag cattgtggtg ccttatgctg cccccgtcac 1800
tgcatttgag ctgatcacaa caaactcagt cagaaccagg ctgaatatgc tgctgtggga 1860
ctcaatcaat caatgggttc ttggagacct ttacttgtct tgcagtttcc ctctggcaac 1920
aacaaggtgc tactcaaatg gaacaatcag tgccatcgct gtcaaagtag agtctgtgcc 1980
aaacctcagc ccaagctggc tgaccttgaa ggaccagtca tgcacaccag cattcattga 2040
tgaccgcttt gctcactttg tcttccatgc tgactcgtgt ggaaccacta gactgttctt 2100
cgacaactac atgatgtacg aaaatgaaat caggctgaac ttcaacagga aaggagttgc 2160

ttacacatca ccagttgatc ctgattacaa gcaaaccatc tcctgctact atgtggtcaa 2220

tgatacccag agaatctcct ttgcttctca accaagactc catgaaccca aagcagagat 2280

tggattcgga cacctggtgg ttcaaatgag actagctcag gatgcttcat atcaaatctt 2340

ctaccaaact gaagactatc cagttcagaa gttcctgagg gagcctctct actttgaagt 2400

ggagctgatg cagtcaagag accctaaact ggaactggtg ctggaaaact gttgggcaac 2460

aagcaaagaa gagaggaact ctctcccaag ctgggacatc attatcaatg gctgtgaaaa 2520

cccagatgac agttatgctg cagtatttca ccctgtaatg aaggacagca gagtttcaat 2580

cccatctcat gtcaagcgct tctccatcat gatgttcgcc ttcatccaga atgaccaagt 2640

tctgaaggat gagattcatg tccactgcaa tgtagtgatc tgtgatgcca acacacctgc 2700

agaaggcatc tgcaaaggcc aatgtggtta ctcatctggc attaaggcac accaaagcac 2760

aaaaaaggga caaagaccac aaagctcaac caaccacaag cagatctcct ctgggcgtat 2820

tctgttgaat aactgaatgt ccaaataaaa atttcttaaa tg 2862

<210> 4
<211> 1278
<212> DNA
<213> Oryzias latipes

<220>
<223> cDNA for gene 4

<400> 4

agtttgtagt cactaacacc ataatggggt ttggcaagtg gttgtttggt gttgtgattt 60

tggcttgtgg tgcgtcagca caaaaccact ggacaccaca gaaacaccag cccctgtttc 120

ctcatcaagc gcagccactg gtcactactt ttgataagtg caatgtggag gagggtgaca 180

agattgaatg tgggacttcg gatatcactg tggagcagtg tgaaaaaatc aactgctgct 240

ttgatgggtg gaagtgtcac tatggaaaag gagtgactgt gcagtgtacc agggatggtc 300

agtttgtggt ggttgttgcc aaagacacca ctgtgcctcc cattgatgtg aactcgatca 360

gtctgctgga gtccaatggt gacttctgtg gtccagttga cagcacctca gcctttgcca 420

tttttcagtt tcctgtgact gcatgtggta ctacactcaa ggacgatgaa aactacattg 480

tctatgaaaa ccacatgtct tcatcatatg aagtaggagt tggacccaga ggatcaatca 540

caagagacag tcattttgag ttgttgttca tgtgcaagta ctctggctca gctgtggaag 600

ctcttatctt ggaggtcaac cctgttcctg ctcctcaacc agttgcagct ctgggacccc 660

tgagagtgga gctcagactg gcaaatggac aatgtctcgc aaagggttgc gtagaagaag 720

aggaagcata caactcattc tacaacccag ctgaatatcc ggtaaccaaa gtgttgaggc 780

aaccagttta tgttgaggtc agggtgcttg gaaggtctga cccaaacatt gtcctaaacc 840

ttgatcactg ttgggccact gcaaccccaa atccccaaag tgttccccag tgggacctcc 900

ttgttgatgg gtgcccttac caggatgaca gatactcaac gatattggtt ccagtggata 960

gctcttctgg ccttgagtac ccaactcatt acaaacggtt catcagcaag atgtttgcat 1020

ttgtggttcc agaaatatat actccccagg agacggtgta catccactgt gccacagttg 1080

tgtgctaccc aagtagcaca aactcctgtg aacaacgctg tcatcgtcag cgaagagctg 1140

cggtaaagat tccttcaagt cagaaggctc tggtctccag tggtaaagtg atcctgatca 1200

agagtcctgc atctcacttg aaaagttaaa attgctcttg gccatctgtt ttaataaagc 1260

ttgtcaaagc tcatgctc 1278

<210> 5
<211> 1003
<212> DNA
<213> Oryzias latipes

<220>
<223> cDNA for gene 5

<400> 5

ctgggatttg gcaatgtggt tctgtttggg ggctgttatg gcagtgaatg cagagctgag 60

gacggactgc aggcctgact acatgtcact agtttggact gacagccggt tgcaggctga 120

tccttccctg tttcgtcttg gtagctgctt tcctgcgact atcagccccc gggaggtggc 180

ttttagcgtg acatatgatg actgtaactt caggagactt gtaactggaa atgagcttaa 240

ctacaccaat gacctcatct acacatcttc tcctgactcg tatgtcaacc cttttagtct 300

cccggttgtc tgctcatttg agaggcccaa ggactggtat cctatgactt atgacccaga 360

gttttccaca tatggtgtag aagacttggt gtttcaagtt ggactaatga atgctgactt 420

cacaggaccc tctgagtcaa atgtgtaccc cctgggctct atgatttctg tcatggctgc 480

tgtggaccag caagaccatc agcctctatt gctgtttctt gaagagtgta tagcctccac 540

cacacctgac cttcaccctg gagccgactt gtatccaata atcacaaata aaggatgcct 600

ggtggatagt aaagtttcgc gttcaaagtt tgaaccaagg gagaaactgt ctgagattca 660

cttgtctgtg caagccttta aatttgcttt gggacatgag gtgttcatcc actgcaccct 720

ggttgcatgg gatccaaatg gactagatga caccaagaag gcctgccact acaacaaaga 780

tcatggctgg gagttgctgg acaaccctgc atataatggc ctttgtgact gctgtgaatc 840

cacctgcaag tcaagaaaaa gaaggaatct gtctgaaaag catggcttgg agaaaaaggc 900

agttgttgga ccactcacaa taacggccta aagtcctgaa ccagcttgtg atttatttta 960

agctgtaaat ccttgaaatg acattaaata aaatttgaaa atg 1003

<210> 6
<211> 1110
<212> DNA
<213> Oryzias latipes

<220>
<223> cDNA for gene 6

<400> 6

tggggacttg catttacctt ggagctgtga tccttgctgt ttttattgca gctggtgctg 60

agccagatat taaagttgtc tgtgcaagag actctgtgaa agttaaatgg agggtttctt 120

ctccctttgt gccgtatgct gctcgtcttt tccttggaag ttgcatgcca tccaagtggc 180

aacttctacc ctctggtgat ggggaggcat tttttgatta caagttctct gaatgcaaat 240

ttactaaaca gataaaagga aaaaatgtag tttataggaa tgaactcagc ttcaggccac 300

aagcaaaggg aaggcccgtt gtttttaagc agccaattga atgtgtctat aagaggcctg 360

```
agggttggat tcctccattc ttgaatcctg gatctggtgt gtctgagggt cacagtaatc 420
tggtttttca catggcgctg ctcaatgaac aactaaccgg tgtggcaaag acaaatgtga 480
tccccttggg ttcattcatg cctatatggg cagcagtgga gcagaagtcc catcagccac 540
ttctgctgct catggatgaa tgtgttgcag ccaccacacc aaaactggat cctggcagcc 600
aggttcacca aattgttgga aatcacggtt gtctccttga aagcaaacga gggagtgcag 660
tgttccttcc acggtaccac tcatctgcca ttatccttta cattcaggcc ttcaactttg 720
gacttggtga tgaggtctac atccactgta atctggttgt atgggatgag ggtgctcttg 780
gccagagtag aaaagcttgc catgtaaaag accatggaag ttgggaactg cttgatgatc 840
catcacgaag ctctgtctgt agctgctgtg actcagtctg cagttcaaag gccaaaagat 900
cagtcggcga gtctggtaca tcaggctaca acacagtgtt gggaccattg gtgataaaag 960
acctgtctgc tgcatctaat gctacactag ttggatctct ccctggaaga agtctgtagc 1020
ggctgaacac gtgaaggaaa agtctctgtc ggagtgaaat ttcagctgta gtctgtatgc 1080
tttaatccaa ataaagtttc tttaaatctt 1110
```

<210> 7
<211> 1258
<212> DNA
<213> Oryzias latipes

<220>
<223> cDNA for gene 7

<400> 7

```
cagttttctt ttaaaccata ttttatcctt acttccatac tggctttctt gtggtgtccc 60
taacacctcc taaggctcgt catgaagaag tttttggctc attcagcctc cctcgtgctg 120
ctcatttctt ttctccgtgg agttgcaatt gccatccgaa ctctaaaaga aggtcctatg 180
attgatgcag acggaaggga atataaaact gcttctttga cagaagactc cagcccgaga 240
gcaagcgata gtgtccgtgt ggagtgcaca gaagtgtcca tgattgtcta catacaagca 300
gacttctaca gaactggacg ccttgtgtct ccaggggact tgtttttggg aggtgcagag 360
```

cataagcagg acagtcggtg tcgggctgtt gtttctggac acaatgagta tgtcattgaa 420

gctggcttgc aagactgtgg ctccaagttg actataacgg atgatgatgt gatctactca 480

aacaagctgg ttttctcacc agtttccaat caccacacta ttacaaggat gactgatgct 540

atagtccctg tgtcctgcca ttacaaaaga acacacactg taagcagcag taatactgaa 600

caggcgccca tgacgttctc tcagtcagca aagttctcaa ccaagaactc tgctttctct 660

ttgaagctga tggctgatga ctggttaacg gagatgttgt ccagcaagtt tcatcttgga 720

gactttctgc gctttgaggc aaagtatacg ggcccagagc ccaggcagct tttgtcgaa 780

agctgcgttg ccactctgac acctgacgca acgtctgtac ccagatacta cttcattgaa 840

aaccacgggt gcttcactga ttcaaaagcg gggtcagttg cctcattcct acccagatcg 900

agagccaatt tgcttcagtt ccagattgat gcctttctgt ttcgtaatga tttgagaaac 960

actatctaca tcacttgtaa cctgaaggct accctccaaa tgaggaccac cttgactgac 1020

aaggcctgca attatgtgca ttcaagctgg aaaagtgtgg atgagaacga tagtgtttgt 1080

tggtgttgtg acagtatttg ctaccaaagt cttcccagag atgatgatct ttgtgacatt 1140

gtcactcttg gtccactgag gattatctct aacaagtaaa gagcaagtga caaactgtct 1200

gccagaggct cttggattaa acctgcgcaa cggtcttgta tgtccaaaaa taaagttt 1258

<210> 8
<211> 1509
<212> DNA
<213> Oryzias latipes

<220>
<223> cDNA for gene 8

<400> 8

gcctcgtcgc tccttccaaa ctctgaggat ggactgcaac ctgcggctgg ccgtttcttg 60

ctggatcatg gtcttttctt gggtttcccc tcttacagag agtcgccaga cgaacagccg 120

aggttctaca gaaaggcact tccaacctcc agtcgggacg cacggcggtc tgcagcctcg 180

gctctactcg gtgaagcagc agccggcccc ggaggtaccc gaacagcacc gccccgtcac 240

ggtcatatgc cacccggatt ccatggaggt tgtggtgaag gccgacatgt ttgaaacggg 300

cctgaatgtg gacggtggac atctgcgact gggttccaac actctgggcg cgggcggtga 360

gtgcggggcg gtccagaaag gagaggacga attcaccatc tgggccctgt tgtccgactg 420

cggaaccaaa ctctcatcaa cagaagagaa gatcatttat tccaacgttc tgatctactc 480

acccgaacct tctgctgatg ggttgttaag attggaagct gcaactattc cagttgaatg 540

tcattatgac aggagatact ctgttgatgg catttccctt gaatcaactt gggttccctc 600

tgtctccaca acttctgtga acgaccagat agatttcaat ctgaaactca tgactggtga 660

ctggcagtct gagagggagt cttacacata tttcctggct gatcccatta attttgaagt 720

ctctgccata gtggaaaatc acgtccctct gcgggtgtat gtggaccact gtgttgctac 780

ggcaactcct gatgcagagg ctaatttaag atatgaattt attgaacata agggctgcct 840

cgttgatgct taccttacaa actccggcgc acgtttccta ccaagaactg aggaacataa 900

actgaggttt cagctggaag ccttcaggtt ctatcaagaa cccagcaacc agatctacat 960

tacttgtgct gtgaaggctg ttcctgctgt acaggccgtc agttctcaga accgagcctg 1020

ctcctttatt gagaacagat ggcaatccat agacggtggt gatcaggtgt gcagaagctg 1080

tgacgtgttc aggcggggtc aggaaccgca agctgtgcca tctcctaaaa tgccagtgaa 1140

cgccaaagac caaatcggtc tttcacagaa aaatatagtc cacaataaag ccgagcatca 1200

accggcttct tacgtccatt tttggccggg agcttatcag agccatcact ccaaacctca 1260

gcagtccaac agatttatga agagggatgc tgacaacaaa tttcatcaag ctgtccaact 1320

ggggcccctc gttgtgctac cgtcaagaaa agtagtttca gtggcaacaa atttttctac 1380

atggtcagaa aagaacaaca cctcctgaga cctggaacac ctgatggagg agtctgagtt 1440

cgtttgagag atttggtctt gttgggctaa agaattttgc tgcaatccaa taaattaagt 1500

ttctaatgt 1509

<210> 9
<211> 1570
<212> DNA
<213> Oryzias latipes

<220>
<223> cDNA for gene 9

<400> 9

cgcggccgct gggtcagcga ggagcacttg cagtctgatt tttgggtgct gctgcttcag 60
tcattttgat ggctccctca cggttaaaga ttagctgcct ttttgggcat ttgactgcct 120
tctgtcttca gttgacactt gcgtttccac cattgcttta cgtgccgcct gtttctctca 180
aaagccaaag ctccttgccg agtgtggtcc agcagccaga ggagccggtg cccgttaaca 240
cagttggtgt gctctgccac cctgattcca tggagctcag catcaatgct gacctgtttg 300
aagtgggagc gcctgttgac gtccgtgagc tgcgtctcgg agtcgagcac agcgactact 360
gcagtgcaac agcatcatca gactctgagt acagaatcct cgtgggcctg gaggactgtg 420
gcaccaaaca ctggatgaca gaagactctc tggtctacac aaacctcctg atttatactc 480
cccttcctgc acttaatgga ataactcgaa tggaggaggc cgttattcca attgaatgcc 540
aatataaaag gaagtacagt ttatccagct catcactcgt gccaacttgg gttcccttca 600
cgtccacaca agctgctgtg gaaactctcc agttcaacct gaggctcatg accaatgact 660
gtgccataa ctgtaaagaa acctcagagg ccaaagaaat cctggcagaa acgtctgaat 900
ggacacaacc agccctttgt ggaggaaaac ctgcagcggc tcttcgctct gcgcatgcga 960
atctccagac gtgccaaagt ggaaaccaac cttgccggtc tcttcaacga gcgcaagatc 1020
cctcatttcg ttgacccaga ggtcaacctg cgtaacctgt ttggcatcaa acctcccatt 1080
cctttggaaa gtcctgctgt ggcaccagtt aaatgaaaat tcagatctga tgagttattt 1140
taggaagttg ggtcttttta gtttaaattg ggcatttatg ttcaaagaat gttaaaattt 1200
ggctgcacga aaggggtgcc aacacgttct tcctcggcga gccaatcaac attgaggcat 720
cagtcagggt ggatcatcac atggggctcc ggctgttcct gaacagctgc gtggccacac 780
ttgaacccaa catcaaatct gatcccaaat atgtctttat tgagaatggg tgcctgctgg 840
actcccagct tccaggctca aagggtcact tcctgccaag aacaaaagac aatatactgc 900
agatgactat cgattctttt aaattccata atgacgaacg aggacagctt tacatcacat 960
gtcatctcaa tgcggtgcca gtggatgatg cagaagcaca aagcaaggcc tgcacatatg 1020
taaatggaag gtggcggtca gctgatggaa acgactacct gtgtggattc tgtcaaagcc 1080
ccaatgaagc cagtaaagca ctcagcatcc ctggagtctt taatcctcgt agctttggaa 1140
aatctgcaga catggagccc atgtggagaa gtggactcaa gactaataag gtttgggaac 1200

atgaagccag actggggcca gtgactattc tgccttcatg gaagagtggg gctcttgctg 1260
cacatgaact acctccagtt ctccataaga tccacaaaac ggcgctgtac ggcagccact 1320
ggaggagcgg cctaaacaca atcgagaaga gcctggttcc agaaccatca agttcagaac 1380
tagaagagga tgacagtgat gactatgatg agtctgactc tgacctggcg tttgtgatga 1440
agtccttgga gattgcccat accaacacta ccttccccat cgtcacatat gacctggagc 1500
cctctaagat gaatgcaact gctgctaacc ctgacctttc tgataaacat gacccaaaga 1560
aataaaatgc 1570

<210> 10
<211> 1278
<212> DNA
<213> Oryzias latipes

<220>
<223> cDNA for gene 10

<400> 10

gaagattaga tgatgaaagc tgagaaagct gttggggctg ggccccagca ggtgttcacc 60
tgcacccacg ccggatgtgg agcctgcttc ccgagggagt ggaaactaaa ggcgcacgaa 120
actgtgcaca ccggagagcg tccttgcgcg tgcccaacag ccgggtgtgg tagtctcttc 180
aagagaacat cccatctgaa aagacacgtg ctccagcata aaggagtcaa agggttccaa 240
tgcaagtttg caaattgcgc aaagagtttc atcgacgctc aaaggctgaa gaagcaccag 300
aacagcgctc atgggaatca caaattcaag tgtaatcaac ccaagtgctc cttgagcttc 360
aagaagcgca gattgttgaa gctgcattta aaggagcata atgtccatcc gaacttcaaa 420
tgttctaaca ttaggtgtac tgcaacgttt gactcccata ttgcacgcaa agcccatgag 480
aagaagcatg caggttatag ttgccctcac aaagactgcc aggttgttga acacacctgg 540
agcaaacttc agaggcacct ggccaaacac ccagtctcat ttacctgtgg cgtgtgcgag 600
aaggtgtacg acaaagcagg tgctctgcgg cggcacaaac ggatccacgc ttcccataaa 660

cctgtgctgc tgtgtccaag agctaactgc caggcctact tcaccacgac ctttaaccta 720

gagcatcaca ttcgcaaagt gcatctccag ctcctgaagt ataaatgttt cttccccgac 780

tgcccacgca catttgttat gcgggagagc atgcatcgac acatggttca tcatgatcca 840

aaatttcctt aaagtaaaag gctaaataaa aatgctctgt agtgttattg acccaataaa 1260

atgagttttg ttttgctc 1278

<210> 11
<211> 1557
<212> DNA
<213> Oryzias latipes

<220>
<223> cDNA for gene 11

<400> 11

ggaaagcttc agttggtaag acgtcagtgg ctcacattca cctacaacca tggcgaagga 60

gaaggttcac gtcaacgtgg tcgtcatcgg tcacgttgac agcggtaaat ccaccaccac 120

tggacacctg gtctacaagt gtggaggcat tgatcccaga aagctggaga aatttgagaa 180

ggctgcagct cagttgggaa agagttcctt caagtttgcc tgggtgctgg ataagctcaa 240

agctgagagg gagcgaggga tcaccattga tatctcactt ttgaaattta acactcaaaa 300

gtacaccatg actataattg atgctccagg gcacagggac ttcatcaaga acatgataac 360

tgggacgtcg caggcagatg tggccctcct gatggtctcg gcagccaaag gggagtatga 420

agccggcgtt tccaggagcg gtcagaccag agagcacgcc ctgctggcct acacgctggg 480

ggttaagcaa atcatcgtct gcgtgaacaa gatggatctg accgagcccc cttacagcca 540

gaaacgctat gaggaagtga tgcgcggcgt gagcggcttc ctgaggaaga tcggctacga 600

cacgaatgcc gtgcccttcg ttccagtttc tggatggact ggggagaaca tgatcagcgt 660

aacccagaag atgccctggt accaaggctg gaaaatcagg cggagggaag ggccttcaac 720

tgggaagact cttcttgaag ttctggactc tattcagcct ccggtgcgaa caatcaacaa 780

acctctacgg ctacctctgc aagacgtcta caaaattgga ggagttggga ctgtgccagt 840

ggggaagatt gaaacaggcg tcctcaaacc cggcatgacc ttggtgttct ctccagctaa 900

gctcaccgca gaggtcaagt caattgagat gcaccaccag ggcctgcaga cggctctgcc 960

ggggcacaac gtcgggttca acatcaaaaa cgtgtcagtc aagaacctgc gccgtgggga 1020

cgtggccggc aacgcccaac aggatccacc ttcagatgtc cgcagctttg aagcacaggt 1080

gattatcctg aaccatcctg ggaagatcaa ggcgggatac tctcctgtcc ttgactgcca 1140

cacgacacat gtcacatgcc gcttcaccga gctgaaggag aagctggacc ggcgcaccgg 1200

caagaagctg gaggagcagc cacaaaccct ggtgtctgga gatgctgcca ctgtcaaact 1260

ggttcccgtg aagcccatgt gtgtggagag cttcttcaca taccctcctt taggccgctt 1320

tgcggcaaga gatctgaagc agacagttgc tgtaggagtc atcaagtcgg tggagaaaga 1380

ccaggggtct aaagctcaaa agcttcaagt ttgtaaataa gtgctctgta aatgtatgct 1440

gcgaaaaagc tttttatttt ccagtgaatt tcagtttgtt taaaatgtat aatttgacca 1500

gaaaagatgc agctggtata tttctgttaa tttaaacaaa taaaaactgg tttggag 1557

<210> 12
<211> 865
<212> DNA
<213> Oryzias latipes

<220>
<223> cDNA for gene 12

<400> 12

tcattgttca cagttgacgg gctgctttaa gatggcaaag aaggtgctga tcgtgtatgc 60

ccaccagagc tctagttcat tcaattctgc agcaaaaact actgctgtgg aagttttgac 120

cactctgggc tgctctgtgg aagtttctga cctgtatgct atgaatttta aagcaactgc 180

cactgctgag gacattaaag gtgaccttaa ggatgctgaa aattttagct acttggatga 240

aagtaagctg gcatgggagg aagggagact aatggatgac atcactaagg agcagtctaa 300

ggtcattgag gccgacttca tcatctttca gtttccaatg tactggttca gtgttcctgc 360

catcatgaag ggctggattg accgtgtgct cacaaacggc tttgccttca cacaagagaa 420

acgttacagc cagggaatct tcaaggaaaa gagagccatg ctgtccttca ccactgggtc 480

acttgaatca atgttcagtg ctactggcat taatggagac atgaatgtca cgctgtggcc 540

gctgcagaat ggaatcctgc actactgtgg cttccaggtt ctggcccctc aaatcttctg 600

ggctccattc tcagcaaccc ctgaagctcg tagctgcatg ttggagggct ggcgtgcacg 660

actgcaaggc cttctagagg agcagcctct gtcattcatt tccctggact gctttgacaa 720

aaaggggttc caattgaaat ctgatgtcca ggagaagcac gcagccaagg actttggtct 780

ggcggtggga atccacatgg gaaagcctct gccacctcac aaccagatga aagctggatc 840

ttaaacatgt ttgcttgaat gatga 865

<210> 13
<211> 705
<212> DNA
<213> Oryzias latipes

<220>
<223> cDNA for gene 13

<400> 13

cttttgggtt ctagtttagt ggtaactgta gcagaatgag gtttctgtgg atttcttgtc 60

ttctcattgg aagcatctcc tgtcttcctc aaggaggata tgatccatcc ctgtttctat 120

acactggaca agctccatcc tatgagaaac cttctgcaca gtccagtggc tacagcagtc 180

cacaaggcta ttacagtgct ggcaccaaca ctgctggagg ctctacagac agtactgctc 240

ccatgtggta ctctgcttcc tatcctgaac aagagccagc caagccaact tatcagagac 300

ctgcacagtc cagtggttat ggcagctact ctggttctgg atctcaacag tctggttccc 360

agggcgctca gtctggagtt ccaggcagcc agcaccaggt tgaacaggag agctggagct 420

cctcatctga cgacgaggaa gagcccgagt tcactccagt gagtgaggag gatcaagtgt 480

acgctttcaa gtctcgctct cgctacaacc agaaacggct gctgttcagt cagttccgct 540

acaccccaac agaaccccgt gttcctcaag aaccagtgtt cccgtacccc ggcaagtctc 600

atcagggcaa aggttcagct aaaggacgcc gctaagatct ggcttttgtt ttgaggaaac 660

cgactgattt attctgaaga ataaattaaa atcttaaaat gttac 705

<210> 14
<211> 725
<212> DNA
<213> Oryzias latipes

<220>
<223> cDNA for gene 14

<400> 14

ctgaggtttc tgttctttgg gggtggagca gctgcagaaa aatgaggttt ctgtggattt 60

cttgtctgct cattggaagc atctcctgtc ttcctcaagg aggatacgat ccatccatgt 120

ttctatacac tggacaagct ccatcctatg agaaaccttc tgcacagtcc agtggctaca 180

gcagtccaca aggctattac agtgctggca ccaacactgc tggaggctct acggacaata 240

ttgctcccat gtggtactct gcttcctatc ctgaacaaga gccagccaag ccaacttatc 300

agagacctgc acagtccagt ggttatggca gctactctca acaatctggt tcccagggtg 360

ctcagtctgg agttccaggc agccagcacc aggttgaaca ggagagctgg agctcctcat 420

ctgacgacga ggaagagcca gagttcactc cagtgagtga ggaggatcaa gtgtacgctt 480

tcaagtctcg ctctcgctac aaccagaaac ggctgctgtt cagtcagttc cgctacaccc 540

caacagaact gcgtgttcct caagaagcag tgttcccata ccccagcaag tctcatcagg 600

gcaaaggttc agccaaagga agccgctaaa gatctgactc catgtgttct gtggctgttg 660

gcaaatacct aaatgcaagt gctgtgtggt ctttaaaata aatatttaaa gttcgactgt 720

cgtgg 725

<210> 15
<211> 728
<212> DNA
<213> Oryzias latipes

<220>
<223> cDNA for gene 15

<400> 15

ctgtctcttg tgtataatcc agcagcattc acatcatgat gaggctcttg ttactttcat 60
gtttcctcct tggacgtatc acctgctatc ctcaacaagg tagtggtggt cattttctcc 120
cataccaagg tcaggctcca tcctatgaaa aaccattgat gcagtctggc tacagtggct 180
ttcctggtgt atacagcagc agcatgaaca cagctggagg cagtggaagt cctcccatgt 240
ggtactctgc ttcctatcct gaacaagagc cagccaagcc aacttatcag agaccagcac 300
agtccagtgg ttatggcagc tacggcagtg ttgacagcag ctactctggt tctggatctc 360
aacagtctgg ttcccagggt gctcagtctg gagctccagg cagccagcac caggttgaac 420
aggagagctg gagctcctca tctgacgacg aggaggagcc agagttcact ccagtgagcg 480
aggaggatca agtgtacgct tccaagactc gctctcgcta caaccagaaa cggctgctgt 540
tcagtcagtt ccgctacacc ccaacagaac cccgtgttcc tcaagaacca gtgttcccgt 600
accccagcaa gtctcatcag ggcaaaggtt cagccaaagg aagccgctaa gatttgtgag 660
ggccactgaa gatcacctga tttgactttt gtaatctaca gactccgcta ataaatgaat 720
taaaattc 728

<210> 16
<211> 729
<212> DNA
<213> Oryzias latipes

<220>
<223> cDNA for gene 16

<400> 16

ggcacgagat aatccagcag cattcacatc atgatgaggc tcttgttact ttcatgtttc 60

ctccttggaa gtatcacctg ctatcctcaa caaggtagtg gtggtcattt tctcccatac 120
caaggtcagg ctccatccta tgaaaaacca ttgatgcagt ctggctacag tggctttcct 180
ggtgtttaca gcagcagcat gaacacagct ggaggcagtg gaagtcctcc tatgtggtac 240
tctgcttcct atcctgaaca agagccagcc aagccaactt atcagagacc agcacagtcc 300
agtggttatg gcagctacag tggtgttgac agcagctact ctggttctgg atctcaacag 360
tctggttccc aggggctca gtctggagct ccaggcagcc agcaccaggt tgaacaggag 420
agctggcgct cctcatctga cgacgaggaa gagccagagt tcactccagt gagcgaggag 480
gatcaagtgt acgcttccaa gtctcgctct cgctacaacc agaaacgact gctgttcagt 540
cagttccgct acaccccaac agaaccccgt gttcctcaag aaccagtgtt cccatacccc 600
agcaagtctc atcagggcaa agtttcagcc aaaggaagac gctaagatct gtgagggcca 660
ctgaagatca cctgatttga cttttgtcac ctactgactc tgagctaata aatgaattaa 720
aattccctc 729

<210> 17
<211>
<212> DNA
<213> Oryzias latipes

<220>
<223> cDNA for gene 17

<400> 17

ggcacgagag ggtgctgttg gtttgtctgc ttgttggaac tgttacctgt gttcctcaag 60
gaggtggagc ttatcagccc agagggcatc ctccattctc tggacaactt cagtctgagc 120
cagtctatga aaggccttcc ggacagtcgg gttatagtgg cgctccggga tattttacca 180
gtggaaccta cactacagga ggcagtggaa gtcctcccat gtggtactct gcttcccatc 240
ctgaacaaga gccagccaag ccaacttatc agagaccagc acagtccagt ggttatggca 300
gctacggcag tgttgacagc agctactctg gttctggatc tcaacagtct ggttcccagg 360
gggctcagtc tggagctcca ggcagccagc accaggttga acaggagagc tggagctcct 420

catctaacga cgaggacgag ccagagttca ctccagtgag cgaggaggat caagtgtacg 480

cttccaagac tcgctctcgc tacaaccaga aacggctgct gttcagtcag ttccgctaca 540

ccccaacaga accccgtgtt cctcaagaac cagtgttccc ataccccagc aagtctcatc 600

agggcaaagg ttcagccaaa ggaagccgct aggatctcgt tgagcgtcac tcatgatgtt 660

ttgatgttga gctgctgaaa agaataaaaa aaatac 696

<210> 18
<211> 731
<212> DNA
<213> Oryzias latipes

<220>
<223> cDNA for gene 18

<400> 18

cttgggcatc agtcaatagc aaccagcaga atgagggtgt tgtggatttg tctcctgatg 60

attggaagca tcaactgcct tccccaagga agtgtcccaa atatggcagt ccctcgctcc 120

atgtggcttc ctccttacta tgggcaagaa ccatctagac catcctatga agagccttct 180

ggacagtatg gtggttatcc caccttccca ggatcttaca gcccggagcc tcaaactggg 240

ggcagtggaa gtcctcccat gtggtactct gcttcctatc ctgaacaaga gccagccaag 300

ccaacttatc agagaccagc acagtccagt ggtcacagca gctacggtgg tgttgacagc 360

agctactctg gttctggatc tcaacactct ggttcccagg gcgctcagtc tggagctcca 420

ggcagccagc accaggttga acaggagagc tggagctcct catctgacga cgaggacgag 480

ccagagttca ctccagtgag cgaggaggat caagtgtacg cttccaagac tcgctctcgc 540

tacaaccaga aacggctgct gttcagtcag ttccgctaca ccccaacaga accccgtgtt 600

cctcaagaac cagtgttccc ataccccagc aagtcacatc agggcaaagg ttcagccaaa 660

ggaagccgct aagatctgtg gtctcctggg ttactgatat tttcaaatgt gaaattaaag 720

tttcctctga c 731

<210> 19
<211> 761
<212> DNA
<213> Oryzias latipes

<220>
<223> cDNA for gene 19

<400> 19

tgttttgttt tgggtattag tcagtattat ccagcagaat gagcagggtg ttgtggattt 60
gtctcctgat gattggaagc atcaactgcc ttccccaagg aggtgtccca aatatggcag 120
tccctcgctc catgtggctt cctccttact atgggcaaga accatctaga ccatcctatg 180
aagagccttc tggacagtac ggtggttatc ccaccttccc aggatcttac agcccggagc 240
ctcaaactgg gggcagtgga agtcctccca tgtggtactc tgcttcctat cctgaacaag 300
agccagccaa gccaacttat cagagaccag cacagtccag tggtcacggc agctatggtg 360
gtgttgacag cagctactct ggttctggat ctcaacactc tggttcccag ggcgctcagt 420
ctggagctcc aggcagccag caccaggttg aacaggagag ctggagctcc tcatctgacg 480
acgaggacga gccagagttc actccagtga gcgaggagga tcaagtgtac gctttcaaga 540
ctcgctctcg ctacaaccag aaacggctgc tgttcagtca gttccgctac accccaacag 600
aaccccgtgt tcctcaagaa ccagtgttcc cgtaccccag caagtctcat cagggcaaaa 660
gttcagccaa gggcagccgc taggatctgt catttcagga tcattaatcc atgactgctg 720
tgcaggtttt catgtaccag tctaataaaa taccattcct g 761

<210> 20
<211> 629
<212> DNA
<213> Oryzias latipes

<220>
<223> cDNA for gene 20

<400> 20

tacgactgca gtgaaacttt tctagtttaa tttagcaggt tgctgctttt caaagaccaa 60

cgtgatgctt cagtctgtta tgaggttgtt ctatatcagc cttttcctct tatactttgg 120

agcctgtgtt ccacttaaaa aaagtgaaac ttctctcggc tctggcttca gttattccag 180

tcctgggttt ggctctgact actcgggacg cggttcttcc atttttggtt atgactctcg 240

tggcgatttt ggttccggct cgccgagaaa gcaggctgca ggctttgatc gtttcattgc 300

agaaatcttg agcctgagac cttctcgttc tttcccacgc cgtgcctgga cctccaacca 360

ggtgcctctc ggcatggttg agccacgccc cgtgtaccct tcatcccacg ttgtcagaac 420

gagcaatggc taccagcgag ctcgggactt ccggagtgat gccaagtacg ctcaagatat 480

ttttgaccac atagatgagg acggtcaaca agagggccac caaccgactg gaccaacggg 540

tcaaaagacc tactgaggta aaggtgaaga atctactacc tggacagaca gacatacata 600

caaacaaaca aataaatgtt aatctgtct 629

<210> 21
<211>
<212> DNA
<213> Oryzias latipes

<220>
<223> cDNA for gene 21

<400> 21

gtttaaggag ggcagtgaat tcccaaagct tgtgttggag ctgcaggcag gagccatggc 60

tgctgggttc ctgattagca ggtttctgct gatttttgtg ttgagtgaac taaagtactc 120

atcggttttt ccatcagttg ggagttgggg gagttttcag gtaacgttcg accctggctt 180

tcaccatcac cacaaaccca ctaatccatt gatgaattac tggttaaaac ttaaggagtt 240

gccaaatctt tggcaccaca cacggaacaa accgctgtgt tgtgatggtg actcaaaggt 300

gcctcgccat cctgtcaagc ctccagtccc aatctgtgaa ccaacaaacc agggaccaga 360

ctgtcctgtg aaacacggcc caacccatcc tgaaccaaag tggcccatcg tcagtcatgg 420

tccatcccat caccacttgc actggccttt ctttcgtgtc ctgcatggac ttctctgtca 480

ccagcaccct tgtcctcatg cccacagcca cgcctacgat gatgaccgct gttctgcaca 540

tcagcatcct cgccactgtg gaaaacacaa acaccaccat gggcctgtat tctaccaccc 600

tcaccatggg cctggacatc atcatcatca tcatcaccac aaccaccagc agcaggtcaa 660

ggtccccccg catggctgtg aacctcacag taaattatgt tcatgatgtc caagaagtga 720

ttcaagcttt gttggctcct ctgagtcaaa gcagcctgac ctccacggag gactctactt 780

ccaggttggg ccaggaatac tgagctgtgc aggtgtctgc atcaataaag atttctgata 840

gag 843

<210> 22
<211> 13502
<212> DNA
<213> Oryzias latipes

<220>
<223> Genomic DNA for gene 1

<400> 22

gatcttaaac ccgagcccga gtcagaaccc gacccgacgt ggggcactaa atgacaatca 60

ttacgttcgg gtcggttcgg gccgggcttc tctctcgttg actttttaa taaatatatt 120

gtaattgctg caaagaagct ccttaggcgc gcgttttcaa acaactattt attaatcctg 180

tatgtcagaa cggttaccgc acgaaccgaa aggcacagcc agcttcttct tactcagagg 240

gagaacgttc accgaacgca cacacacaca cacacaggta gaccccgccc cctctatccc 300

accagtcacc agcccgccgt tgaatgacac acactgtggt ccagcaactg gcagaaagag 360

gggggcgccg gccacccgca gctctgcgca cgtgtcgtga atgtaatctt ccctccgcaa 420

gcccaaatct attttttaa ggtatccccg atatggagca gcaagaagtc aaggataaac 480

ttaagacagg ggaactgaaa ggcaaacacg caccgcggct gcagaatttc tccgcgtaat 540

gaactttcac tctctttgtc tggcctctaa ttcgagtaag ccctggctgc agaattgctc 600
aacatagtgg aatgctggaa cgctgaggaa tgtgaacacg ctgcattcca ctatgttgtg 660
caattctgca ggctgcagcc aggggtagtt gtcttattct gtgttccagc gttatttcgt 720
tgtgcaaatg catttatcat gtagggagga atctatgcgc agcgcttccg gcggaactct 780
atgccacaac agcgagcttg actacgcgcg cggctgcagc gaccactctc tgttcgaaag 840
aaaataactt tcatgatcat aaaaatatgt agattattta acctttaaga aatctggcgt 900
tttttctgc caaaaatact atgggataaa ttaaactttc gggtttgctt cgagctcggg 960
cctgcaaatc aagttaattg gtcgggttcg gaccgggttc ggctttaatg cccgtgggcc 1020
tgtctcgggt cgggctggat tctttcggtc cgatcttacc tctaagctga actcacagtc 1080
tttggttcag agggttaact gcaccacagg caaagcagca atgaggagag tggatgcaga 1140
tttaatccaa aaaagagaaa agacaaagta agtttttaat taaataagga acagaaacta 1200
aaattcataa cagaagctga ctagtaccta aacatcctca tgaagaatgt tttgatttat 1260
tttggtaatg aaattgcttt tgtaactgaa gttgctgttt aagtgaactt aaaaaaccct 1320
ggttatccta tctgcttata catttctaat gctgttaatt tattcctttc tttcatctat 1380
tgttatcaca ctagttttca taattacttg aaacctttgt acttggcttt gctaaaaaaa 1440
aaaaaaaaac atttcaggga attattcttt tttgaatctt caatacttca atacttcaat 1500
gtttcaagca gtagcatttg tttttctttc ttgttgcatc aagtggcctc ttggcaaaaa 1560
agtcaatttt ataccaacca aatgtcaatg cagggcaatg agctctgaaa tctgagtttt 1620
agattgaagc aaaacaagaa atatgttcca gtttagaaac ctccaaggca acaaactcaa 1680
gtgtcaacca caaagaaaaa gtataaggca gaaaaaaact gttctataaa tgtcttgaag 1740
tggcaaattt gccacaacag cccccattat tctaatttat ttgtgtcctg acattcatgg 1800
aaacaggttg tttacttcag cataaggatt actgcacaga aattgatgat acattcacac 1860
aacataaagt ttgtgtcgaa tggcttttgt gtaaaggggt tgttttttc ttgtgtttct 1920
ttcaagtggg ccggtaaaac ataatgtaat tgcatactgt ttagataatc aagtgtaatt 1980
atgccaggca tcctgacgtt cttttgagga gttcaactat acctggaaat tatggcggga 2040
gaaaatgttc tttgtatatg tgtccctgac ctaaactgga gtgttctgca acacaatctc 2100
aatcaaatca agagatgagg agaaagtttg ctgctagtct aaatctgcaa catatacaca 2160
gtacatctga catactcaag ctagccagtt tttcaaagtc catcaaaatg tttaaatgtg 2220
accctgcgtc attttcctga ttggtatgat tcagggtttt gagaagtctc aaatttaaaa 2280

aatctttagt aaaatgcagt ttatttgtgt ttctctttgt gcctctgaaa gattcacatg 2340
tacttaagtc acaggaaatg gtgctgcaaa aataagagta tttccccagc ctggtcacac 2400
ttctgtggga tggcatcctg ctcttcaact acccttagcc cactaggcac ggacatatct 2460
attggatatc taaattatgg ctattttaaa gctcacagct atagatatga attatgcatc 2520
tgacagacat caattcatag aggtctattg catatccggg tttagatctc tagcaaacga 2580
ctttcccgca gctaatcgtg tcacggtccc atcatgcttg tgcattgtaa acaagccgtt 2640
tgggtaccat agtttcttcc cactggacac agaatgtcta gacatctatt gtagatgtct 2700
actggacatc tcagacaact ctattttgtt acactcagat gcctactaat attgttctgc 2760
atacagaggt ctcttatcta cctgtagaca tcaaaccttg gatatttttt agatgtctat 2820
tacagaactt tttttttaa tttatttact ggatataaaa ttgaaaaccc agactgaaca 2880
tgtttctatc agtgagattg tggtgtgctg tcattctttt tatctttttg ttatcaaata 2940
taaatataac aacattggtg tagagtccac tttttgtcct aatatataaa cacatctgtt 3000
ttctaaacca gttttatcct ttacagggtc acatggctgc cagagcttaa cctgacaact 3060
gatgggcgaa gccagggtac accctggaca tgttgccagt ctgttggcca aaaacagtct 3120
gtgcagggcc acaatcacat acaaataggg gcaaattagc atgatcaatt caatcaagtc 3180
tgtattagca tgtggttaac aaatcccaac agtggagcaa catctcaccc atgtggtatc 3240
aaacagtctt ctttttactg cttagatttt aattattggt tttaaatttt gtttcattta 3300
ttattggttt ttcatttttg tgtatgcagt tatatctgtt tgctcattag ttaaataggt 3360
gtcttactta acttttgatt tctattccct ctcgatcatc ttccctagtc tggaaaagac 3420
agacctgaaa gaggtaaagc ttatgtaatg tactttgagt tgcgaagtag ctctgtcttg 3480
ggctaactag agtgtacatg taaatgtacc taataaagtg atttgtaagg tacgctcact 3540
cagttcgtga acacactccc tcagttatct ttgcgcttgt tcaattaatg gcagtcatga 3600
aacgccatag tcccaactga gatttgaacc ggaccttcac gctgtgaggc aatagcgctt 3660
ttcagtagaa atgtttgcta cttcttgtaa tgcagaactt tgtgattgct agttgtctgc 3720
catcaaaaag gctgtatggc ggtgtagagg ttagctcttt caacttaaag taagaagaag 3780
aacctggttc aaatctctgt tggtaccttt ttgtgtgaag tttggacgtt tttcctctgc 3840
agatgtggat tttctcctgc ttactcccac agttcattaa taggtgtcat agtataactg 3900
gttcctgcaa attgctcctg tgtgttttta tggccctttg acaaactggt gacatgatta 3960
aggtgtactc cacccacttc ctgggttgga accagcaaac tggtgactcc aaaagagatt 4020

cagagggcct gaagatgaaa ggatggatgc aaaattgcag tggttatttt taaataataa 4080
tcccttaatg gacatccatg tgcaggagac ctctatatgc agatatttct tagaaaacta 4140
ttgttagaca tctaacagag tatctagaca tcctgtggct agcgggattt accagtcagc 4200
cagcatgttg gtcactgtga aaatgtctga acaccatagt gttcggtgga gttgaggtca 4260
gatctggtgc tacgtcattc tccttcactg cccaaattaa aaggtagtcc tttttataga 4320
gtggttacat gtggttacaa atgaaatttt gctgggacat ataaatctgt gcttatgtgc 4380
ccattcctag tgctccatgc aattgtggaa caaggggagt atcatcttga tattcttcat 4440
tgttcaaata tgtgacgtgc aaaacctgag gtggaaaaac aacagtcaac aaggctgcat 4500
gcgtgcttgt atttgcagat cgaatcatct taattctata gagacaaagt gctctggttg 4560
gctgctttgt gatgattaaa aactgttaaa ttagcagcaa tcagtctgaa caactttgag 4620
ttgagagcaa agacacgcag tgcaaactgg agctcaaaag ccaggttatc agtttattgt 4680
gaagaagtga agacaaactt tgaaactaga tcagacagac tttgtgtcag tgtttgcaca 4740
cgacatcatt tgcaagacat ttacacaaaa catctattaa aaaggtttgt tcatcctcta 4800
gatgacttga ataacactac aaagtggaaa cataaagtga cacttcagtg tcttttcatg 4860
atctgttata acttcagaat taatataaaa cttaatcatc tttcccaaat ggatgcgcct 4920
taaaaatgtg tcacatttta agagaaataa actggttttc caaccatatg agatctatta 4980
tcaaaacgta ttgtttcaac catgaaaaaa tccttttttt ttttggacta taagtcgcac 5040
tttagggaga aatgtacctt acaaaacatt gcagaatgaa tggggatttt atcttgagag 5100
acaaataata aaagaatagg taaaaatgac agactggata tgttcctccc agtgttttcc 5160
cgcactgact gggttgtttg ggtggaaccc gctgccgctt ggacgggttt tattttctgt 5220
tattgggagt gttcaacagg gttttcacgg gtctctttca tccgcttcac tggtccgacg 5280
gatcagccgc tgacagcagc atctcctctc ccacctccct tgtggccgac cacgtgtctg 5340
cgcaggccct cctgggactt ggacgcctgt tgggactcgt gactatctac ccctccctgc 5400
ctacctgcag tagtgactga ttgtactcaa gttcacttgt tgtctttttg tttctttaat 5460
tttgtggtca cactctgcgt gtgagtgggg tattggttct gttttgggtt tgagtaccag 5520
ctctgtttaa aaggtgggct tgaagctcct atagggtttt attttttta ttttaatcct 5580
gtttttaaat ctatttatga taaatctttt aaatttggaa tcacgtctcg acctgattga 5640
gtccccattt ttttattatt gtgcctgaac tgctcgtgtt gccacatatg ctttactact 5700
gtagcatatt gaggcttttt cacgaaacat agaaggaatg tgtcggatct gataaattct 5760

accaaggccg gcatgttttc ttctgcgttg ctgtcagtag caaatagtga tacaaacacc 5820
tacagcgccc tctattggtt tttgctatca caaaaaaagt cccactgggg tataagttgc 5880
atctctggcc aaactatgca aaaaactgtg acttatacac tgaaaaaact agttcttaac 5940
agaatttttt tatttgtgct gatttcatgt ttacccttct aatgttaatt tgtctatgga 6000
catgatggat cggtttttcc ttttatgagt aaaaataatc tctgtttcaa tgtatttact 6060
cttttaaaca cagacctagc tgcactaagg caaagtggtt agcatttttg cctcaaaacg 6120
agaagtcctt ggttcaaatc ccagccagga cctttctatg tgaagtctgt atgttcttct 6180
tttgcatgca ggtcactccg gctttcttcc acagtccaaa aatatgttta atagattaat 6240
tgatgtttct aaattgcccc aaggtctgca tttgggacag gctgcaaacc tgtccaaggt 6300
gtaccctgac tttgctcaac agtctggtac aggctcagac agccctgtga ttccgaaacg 6360
ggtacagtgg gttaaaaaaa tgtgtggatg gatggaaacc cagtcacaca gaaatattgt 6420
aaaatgagaa tagttttcaa gctaacagat cttttgttaa ctatataagt ttacatattg 6480
gtaaattgta gcattttcat ccatttctat aagtgctaca ttcaaatcca catgaattag 6540
aggaggtaag acatagccat agtatagtat tttttgacag ctgaaacacc tgtggataat 6600
caatgaaaat gaaatttaaa aatggtgtct tcctgactgt atttgttttg ttattgtcga 6660
tacgactggt gcgatgacaa atcgcttggc tctttaaagc aagtgacaag agccgcattc 6720
ttcctcttag gaaaagtttt ttttaactgt ccttcactcg ttattacttc tttttctctt 6780
cgttattggt ctaaatgaga taagagccgt ttcgttctcg accgacacat cacagttttg 6840
ttcagtatgc ctttcctgtt gctgaagcca aaaataaaaa tgactggatt tttttctaaa 6900
aaaaaaaata aaagctccaa aggcaggtgt ctcaaatttg atttattttg cgaacaaaat 6960
ttcttttagt agttactttc atcagtttta ttgtatgtgg agtcatgggt catatgtttt 7020
aacctgaggc tagtgggaag ggtgaagggt gggttgggtt tttattgtaa tattgtgtgt 7080
gtgttttttt tttttctttt tctaaatgtt aaagcgcttt gagttacgct atgtatgaga 7140
agcgcttttc tgaaataaag aaataaataa aatcaatctt tatttctata gcacttttca 7200
tataaaacaa aacaaaacac aaagtgcttt acaccagaga aaaaaaaact agccccaacc 7260
cacacagaac ccctaatccc attcaagcct cccacccctt aaatgatgga tataaacatt 7320
tgggaaagag gctgagtatg gacaaaaatt gtttgtgtaa aagttaatat atggaacctt 7380
cctctctgtg aatagctgca tagacagcca tgcagcatca caggtgggga cccagcaaca 7440
ccacagcaag gtggcgatgc aggttcccat ctgagccgca gcggcaaaac agcagcggaa 7500

ccaaagggag aggatccaac tgaggaagct ctggaattta aaatgaagat aaaaataaaa 7560

aataaagaaa acataattag taaaagaaag aaataattaa ccacaaaatg tgaaataagg 7620

aatattcaaa agaattgtta gtcccgtaga aattcatata atttaatata gtggataaat 7680

taataaatac aaaataaata aaaactatta aaatggtaaa attagctaaa agcctgttta 7740

aaaacattag tcttgagcct ttccttaaaa gcaactattc tctctgcagc cctcaggtcc 7800

tctggcagac tgttctataa acaacaacca tagtacttaa aagatgcctc tatgtaggtt 7860

ttgcttttta cagtcggaat gattaattga ctagtcagag gatttcgggt tcaaacacac 7920

gggttcaaac ttaactaaga gatctgacag ataggaaggc acaaaattgt tagtatctta 7980

aagcaggagt gtacatatcc taaaatcaaa aaaactaatc ttcttctgaa tttaaaaaaa 8040

tgtacgtggt tagccttaat ttaattatct caggataccc tcccgaatcc ggtaggtggc 8100

aagattgcac gtggaaactt acaaaatgca aagaaaatta gacaattgcg gcgaagaaga 8160

atcacaaaca gtgggcggag cgcaacgccc ttaactccgc ctacgtgagc cacaactcag 8220

ctcgagccgg tggtgtgagc ggcctaatca aacaaacatg acacaggtgt gctcctcgtg 8280

ccctcagggc tctgttactg atgtaggtat ttgtaaacgg acagctagag ctcagctgaa 8340

aagaagtgta attctattcg aacgtagtct ttaaaaaaaa atgaaggtgc cagaggcgga 8400

attaatgagc gacattctga agaggctgac gggagagtct gctctgccgc tgtactgctg 8460

catcgagaag ttcaagcgcg agaggaacgg cctctacttt gtcgccgagg atttcactga 8520

aaccgtcaaa aaaagagaaa tggtcaacgc caaggaaaga ctgagagtga gttcagttta 8580

gagaccaaaa atgatccata tttctaattt aaaaacatta ttaaacaaag cgaatatcag 8640

acataatatt tgttgtagta tgacagtaat taatgtcatt attgctcagt gcagaaagtt 8700

tgggaacagt agtttcatta tcttcagagc ccctccgggt ccaggaggga cttggccttt 8760

ttctcagtcc tcctttctca tcctgcactc gtctttgaga agccgatcta accatgccgc 8820

gcatttggag cttctgtcta ccatcaacta tgcattgagc aaagacacct ggagaggctt 8880

cagttcaaac attgtgttct tggtgtcaat ttgactggaa agcactacag cttttaattg 8940

ctattttatg tatttaaaac ctacaatttt aaactttata attcagcttt tttgagtgaa 9000

tactcttcca ttaaaaacca gctgtggttc atctttaata attctgaaat tctcaaaact 9060

tttatcaatt tgatctaagt tttgtgtcgc acaatatttc caggctctgc agttatcacc 9120

gagttactgc tggatatgaa atttgggcct actttctcta cagataagga acttgaacac 9180

aatgttctcc cggctgaagc gcatgctgcc tctaatgcaa ccagacaaaa agccaagtaa 9240

agttgataca ctcaaagcag ccactgaata cattcgactt cttcttgctg ttttgcggga 9300
cactgaaaat gtaagactgt cctccggggc ccctgtcaaa tcacaaaata aatgctgcaa 9360
caacaacgct gatatacaat gttgactgct gtcattttac aacttgtaga acaacactgg 9420
gacggatttt ctaaagaatg caatcactta tggtcagcag gatggcttcg ccaatgacct 9480
ctggagaatg gacgatgtga gtatttaaat ctgtggctga aatggtagtt ttaaccaaac 9540
atgtacctta gtagcatcac ctttacatca ggtttagcct ggaaactctg ctccatcgtt 9600
tggaccacat gtgtcaaagt cgaggcccgc aggctacatc cggcccgcca gatgatttta 9660
tattattatt aatggctcag caaagtgtag cgctgataac atatttacta cagatctcac 9720
aatacagcgc ttcagctgcc cgacgaacta taatggtggc atcattttca ctacagatcc 9780
cagaatcagt agcccgcggt ttacatgggc agaatatctt gatcggatca atggtcgggt 9840
taaaatttca ccccgtgcgc aagggatcag aaaacctttt tcccgattag aacaaacgtt 9900
cccatggctc acactttcgg tcggaatgaa tcatttgggc atgcgcagta gtgttaaaaa 9960
ctcccggatg aggaaatggg tcccgttcta aacaaacagc tgccacagac atttatttta 10020
tgctacagct cagtaatata cgagacgatc ttccaaatgt gcttttatta atgtcatgaa 10080
tagtatgaag cgcctgttcg ctcatcgttt tatttaattc gtgtgatcag tgctggaaga 10140
gggttaggac aggctataaa cacaggctaa caacattagc ctgatggaca caaaatccag 10200
gaccataaaa cgctgcaatc tgcattctgg ctgtatgtaa atgtctggga ataacatcag 10260
tctttattta gtcgggacac aactggaaac acaaaaacaa gtgattattt aaacagtttc 10320
taaggactga gcaacattta tttctgcttc ctcaaagccg ctgtgtgtgc tggcggttcc 10380
tcctgagtcc tggagctgct aacccagagc ggcgggacgg cttgcagtct gaattctccc 10440
acacgtaaca aaacaaaaaa aaacgcacac gtaacaaagc atcctcctcc cctcagacac 10500
aaagttatta atccagctcc tctgctcact cgggtgccag tggaccgagt gagcatcggg 10560
ggggcacaaa gcgctccttc cccggatctc cctcatgggg ggtgacagag aggggagagc 10620
cagcggggcg agagcggagc ggcgcttttc acggggcctc cgcagagcag ctggtcggtc 10680
tcgtatgcgc tccaaagctt tctgtcagcg aaacaccatc tatgcgtgac gtaaaccaga 10740
gcagtagtga cacgcgatcg gaatgaccat ttacatgctc cacgatcgga taaacgatca 10800
ggataaccca cttatctcga tcggaaagaa attctgatcc gaacgagtct gatcggggca 10860
gactattccg aacggcgcgt ttacatgacc cattttcttt ccgatcaggc gttctttccc 10920
catgtaaacg cagctattgt tcagctgccc tgctgaacac ttttgctaga cccacgatgc 10980

acaagagaag ttaattctga aaactgagcc tttaaaaact ctttggaggc agaattaatg 11040
tttactgaca ttcagtaaac ctgcatgtca tctgtggagc gaatgtggct gtaatgaaag 11100
aatatactct aagatggtgc tatgagacag atggcaaata cactttttac tgaaaagctg 11160
agcacgtgga gtttgcacag cgctctagta tcattgaaga acaaaaaatg aattttgttt 11220
tgtttgtaac ctgctttccg tcaatgtgga aacggcacct gtaaggattc agatggcgct 11280
gattgagtgt tttggtacac cgaaggcaaa gtacgacctt caggttcaca cctccgttcc 11340
tgcagaaatg ccccagctcc gtctacatgc agcccgatcc ttgtgcatgt ttgggcgcac 11400
gtttctgtgt gagaagctcc tctcagtgat gaaaactaac aaaacagcag acaggagtca 11460
tctccctgat gaacctctac aatacaggac ttcacatcaa acacaaacca acttgacaac 11520
aaatgatgcc aggcgtccac ctgacaaaat gagacaagag caaagaaatt tgactttatt 11580
ttgcagaaaa gcacaaattt tatttatata tccaggtttt atttgttttg ttatgcagca 11640
aatacctatt ttgaattttt gtagttgtga caggatatat ttttatggag aacaaaatat 11700
ttcggtatat ttaagttttt ttctatgaaa tcagagtaaa gttttttttt ttatctttag 11760
tcgttttact ttatttcaaa ggtgtatcat tttgacagtt tatgtttttta tggagagaaa 11820
atataaagta tttaaggttt aagttagctc aacccatata acccagatac acttcttttc 11880
ttaaacatag atatgtggac tacttggacc acagaaccca tttctgatgt ttttgttatg 11940
ctggtgtcac catatgggtg acatcagcat gggttcttgt gggtaatagg atttctgttg 12000
tttagttcag ttattttttt ctgagtttcc actgttagag gaattactga agtcaaatta 12060
cttgcagtca tttctgtttc ctttggttaa caagctgcaa gtgtagttgt cactattact 12120
aaagcagatc tccaaaagca gttggcaaat tctaacaagt ttaattttta caactttctt 12180
atttggtttt cttttagaat ttgactccct taaatctaaa atggatcctc aaattagtat 12240
tttgggggtt ttgttgactg aaaacttatt tggtttgctt acttaacttg cagttcctga 12300
acatctgagc atttaactgt tatttttat ctgtctgaca gttcttgaac ctgtcagatg 12360
atcacatttg gaggatgggt tcaccatgcc agcagaacct gcagcagagg atggagacat 12420
gactagactg gtgttgcagc attgtgtgat gcctgcatac cagttcatca tccaagtagc 12480
gcctgatcaa gcttcggtaa gtaaacaatt aaaggcgtgg gacttgatgt tttaaggtgc 12540
aatttgtttg catgaaatga caacgtatca tttaatcctt caacactgga cctttgagct 12600
ccattgtcta cagaattcat atattttggt ggattctgaa gtggaaaaaa acggctttgt 12660
gtcggtgtgc aactttttac cataatagtg tgtagcacaa agtgcatatc ggcggtgcac 12720

agctaacata caaagccact ttttttctgt ctgaatcagc tgattcacac taaatggttg 12780

aaaggtatgg cagatggtgt gttatgtagg tcttgctggc gatatctcca gtgttggagg 12840

gttaaattgg cctttgaatt cattgaggcc aaaataaaat ttattaaaat taagtcgggc 12900

ctgaactgga ataactagtg aacaaagtaa aaccataatc ttaaaataaa atatgaattt 12960

atatcatttt aatcaattgt gaatgtgttt aaatcttttc agtggcagcc acgaatcaaa 13020

ttctatacag tgatccctcg ctataacaca gtttactttt cacggtatcg ctacttcacg 13080

gatttgcatc gtgcattgag ttctgcattc tgattggcta aaaagtcact ccccttcttc 13140

tacctgtgca tcaataacgt tgcagtttaa tatgtgcacg tacgtaaaac agctcgccaa 13200

atttacatta tgtacgtgca aattttcttt ctggtggcat gtcggtgtat aaggatcttt 13260

tggcaaagaa gaaaaaagag cgacatctgc ctatcactac gttcttctcc ctaacaaaca 13320

cagctgcacc gcgggcttca aaagaagaaa acactgcaga gcggagtcag gatgcagcgg 13380

ctcagtctga agggcagttg aaatacacct gagtcactat ttgtcccgac tggaccttgg 13440

aatttttttc ataatcattt taaaattttc tccagtttaa tcctattact cgggtccgcg 13500

gg 13502

<210> 23
<211> 4067
<212> DNA
<213> Oryzias latipes

<220>
<223> Genomic DNA for gene 8

<400> 23

ttgccaattt tttttattat gttcaaagtt ggaaagaaaa acaaaataca ttttaatagc 60

actttcattt gtgacatgaa aagctagaaa tttacagatt gcgagttatt tcaagtacag 120

gcatgtttta ttgccacctt gtggactttt atggtaatga cctgccaggt ggacctaaac 180

tggttcgatt aatatacagt ccataaaaaa cagtcataat caacaataag agtgaacagt 240

aaacaatcat tggtctcttc tattcgcccc tgtctcgcgg tacatgcgca cttacagggg 300

cggggagggg cccctgattg ctgagctcct acaggtgtgc ttgagcagca ggtttaatcg 360
cctgcagctg ctcggactct tttcagtgcg cagggcctcg tcgctccttc caaactctga 420
ggatggactg caacctgcgg ctggccgttt cttgctggat catggtcttg tcttgggttt 480
cccctctgac agagagtcgc cagacgaaca gccgaggttc tacagaaagg cacttccaac 540
ctccagtcgg gacgcacggc ggtctgcagc ctcggctcta ctcggtgaag cagcagccgg 600
ccccggaggt ccccgaacag caccgccccg tcacggtcat atgccacccg gattccatgg 660
aggttgtggt gaaggccgac atgtttgaaa cgggcctgaa tgtggacggt ggacatctgc 720
gactgggttc caacactctg ggcgcgggcg gtgagtgcgg ggcggtccag aaaggagagg 780
acgaattcac catctgggcc ctgttgtccg actgcggaac caaactctca gtaagtttcg 840
aacacagcga gcatgcgcct aatgagtcca gcatgaaaag actgtcttct tagtcaacag 900
aagagaagat catttattcc aacgttctga tctactcacc cgaaccttct gctgatgggt 960
tgttaagatt ggaagctgca actattccag ttgaatgtca ttatgacagg tgagtcctgc 1020
gtgcatattt atacgcacac ctattttgtg caacagtggc cctatggaag agtgtatgcc 1080
ctgagactag aacgtcgtga gcctcaaatc catgagtcat actggtaccc aaatcctacc 1140
tgcttgactc tcagaatcaa gggattggat tgggggttta aactgccaaa tggttcccaa 1200
taatggctgt ctctgcagct tcccatttcc catgcctggc tgtattgacg actaatggaa 1260
cattaacatt aaattttatt ttctctatag gagatactct gttgatggca tttcccttaa 1320
atcaacttgg gttccctctg tctccacaac ttctgtgaac gaccagatag atttcaatct 1380
gaaactcatg actggtaatc aggggtggct ttggaaatta tattttgtct gttcaaagcc 1440
aatatgcggc ttcaacaccc tgattgtttc aattgcacag gtgactggca gtctgagagg 1500
gagtcttaca catatttcct ggctgatccc attaattttg aagtctctgc catagtggaa 1560
aatcacgtcc ctctgcgggt gtatgtggac cactgtgttg ctacggcaac tcctgatgca 1620
gaggctaatt taagatatga atttattgaa cataaggggt gagcttaaag tcaagcattc 1680
tgaaagttac ttttttttgc ctttattact catctgacat ttctgccaaa ctagctgcct 1740
cgttgatgct taccttacaa actccggagt acgtttccta ccaagaaccg aggaacataa 1800
actgaggttt cagctggaag ccttcaggtt ctatcaagaa cccagcaacc aggtgtggct 1860
ccaaatgaaa catttgtacg cttatgaaag ttttcatact gcttaaccat ttttgtcgtt 1920
gcacaacaca aggtaactcg gacactttgt gaactcaaat cttggcgttc tgcgtagttc 1980
ccccttttggc atatcccttc agggggcgcc acagcaaatc agccttcact gagtcacaca 2040

ggtggtttgg cagagtttta tgccagatgc ccttcagaca accatgcatt tttacctggc 2100
ccatttttat ttgctggtta tagttcagca gtagcgcaaa gggtcttgcc cacggaccca 2160
tgctggatga agcttattgt gttccatggg aattgaaccc tgattccccg tgtgccttaa 2220
gcaattggac gatcagtcac tatagtcggt ttaattattg tgcttgatcc aaaattattg 2280
ctgactggtt tgggaaaact gcttgtgagt caccactttt ccctattgtt tccagattta 2340
cattacttgt gctgtgaagg ctgttcctgc tgtacaggcg gtcagttctc agaaccgagc 2400
ctgctccttt attgagaaca ggtgatctat tgaaatagaa atgcaaaaat gttctagtgc 2460
ttgttgcaca aatcttaact gctaccatct cgctccagat ggcaatccat agacggtggt 2520
gatcaggtgt gcagaagctg tgacgtgtcc aggcggggtc aggaaccgca agctgtgcca 2580
tctcctaaaa tggcagtgaa cgccaaagac caaatcggtc tttcacagaa aaatatagtc 2640
cacaataaag ccgagcatca accggcttct tacgtccatt tttggccggg agcatatcag 2700
agccatcact ccaaacctca gcagtccacc aacagattta tgaagaggga tgctgacaac 2760
aaatttcgtg agtagaactt aaagggccta tttcatgcaa ataaactttt tgagctttta 2820
aattgttagt tcctcacaaa aaacaactcc aaagcagtat tttgctacag tcacgcattt 2880
ctgagcattc ctttaaaaac ctgctctgag caccagcccc tcccaatcca caaaaacaca 2940
ttgtgagcga ggaacagccc cttccatgaa gagtctgcgc tgccagcacc gcccccaggc 3000
taacccacac ctactttata catggagcta gcgttggttg atcggcaaaa acgtttttat 3060
tgtcatatgc tcagttgtct ctgcaaaagt tcagacgttt tcgtgggcga aacagacatg 3120
ctgcggctct aggttgattg tgaaaagggc ggcacccaca cggagtgaaa gtcgttgcat 3180
cagatcgagt attacttctg ggtaggaaaa tgatctgaga aaatgactaa tttcataaat 3240
ttgttttttt ttgagtctgc taaaggttat acatatggat acacttgact ctttaaaggc 3300
ttgataaaag catgaaatgg ccccttcaat taaaataaat ctgaagagtt tgtgttaatt 3360
taatgtttgt ctattgtaaa agctggaaag tatctaaaat tgactcttgg ttttgaaaat 3420
gtcttacttt gcagatcaag ctgtccaact ggggcccctc gttgtgctac cgtcaagaaa 3480
agtagtttca gtggcaacaa atttttcaac atggtcagaa aagaacacct cctgagacct 3540
ggaacacctg atcgaggagt ctgagttcat ttgagaaatt tggtcttgat gggctaaaga 3600
attttgctgc aatccaataa attaataaat ttctaatgta aactttattt gtaaaaatga 3660
tgctgcttat tctgtggacc actctttctc cagtaaatat tactggtttg tgtccccttt 3720
aaatgtatat aatatcgact aaagcaattt gtgtggagaa gctgttggtc catggacttc 3780

```
ataaaactaa agcgaacaac ttcattgtta ttgtatatca ggcacttggc ctcattaaac 3840
ttttggaaca taactagata ttggattatg acaaattgaa gacgactgga gaaacgtgat 3900
cctagctcat gtctcttgca gggacatgct attagggagc atttccttcc attactgcca 3960
cacctaatca tgtagtcaca gtattgatta tctgaaggct atgatgtaac gtgtttgctt 4020
agcttgagtg atcaattaca gctttgcctt aattattctg gaacctt 4067
```

<210> 24
<211> 21
<212> DNA
<213> Artificial Sequence

<220>
<223> PCR primer F1

<400> 24
tccttccctg tttcgtcttg g 21

<210> 25
<211> 21
<212> DNA
<213> Artificial Sequence

<220>
<223> PCR primer R1

<400> 25
ttgcaggtgg attcacagca g 21

<210> 26
<211> 21
<212> DNA
<213> Artificial Sequence

<220>
<223> PCR primer F2

<400> 26
gctttcctgc gactatcagc c 21

<210> 27
<211> 21
<212> DNA
<213> Artificial Sequence

<220>
<223> PCR primer R2

<400> 27
atttggatcc catgcaacca g 21

## Description

### Technical Field

The present invention relates to a method for rapidly assessing an endocrine-disrupting activity of a chemical substance.

### Background Art

Endocrine disruptors (often called environmental hormones) collectively refer to chemical substances released into the environment for which hormone-like activities or anti-hormonal activities have been found. Altered reproductive potential (in particular, conversion of male into female), decreased reproductive potential, decreased hatchability, decreased survival rate of offspring, abnormal reproductive behavior and the like have been reported to be resulted from the influences of endocrine disruptors on the ecosystem of wild animals. Decreased number of sperms, endometriosis, infertility, ovarian cancer, uterine cancer, prostatic cancer and the like have been suspected to be resulted from the influences of endocrine disruptors on human health, although they have not been demonstrated.

Substances (or groups of substances) that are considered to cause endocrine disruption are reported in the interim report (July 1997) by "Exogenous Endocrine Disrupting Chemical Task Force" of Environment Agency. However, it is considered that the types of such substances would be further increased in the course of research and study in the future.

Known methods for determining endocrine disrupting activities are classified into two groups, i.e., in vitro methods and in vivo methods. Examples of the methods in the former group include a method in which an activity of binding to estrogen receptor, androgen receptor or thyroid hormone receptor is measured, and a method in which an activity of inhibiting a hormone synthesis enzyme system is measured. Examples of the methods in the latter group include a method in which production of various hormones and abnormal tissue formation in individuals at different postnatal days are determined, a method in which abnormal metamorphosis in a frog is determined, and a method in which abnormal maturation in a fish is determined (Analytical Chemistry, 70(15):528A-532A (1998)).

The in vitro method is advantageous because it is sensitive, and it can be used to assay a number of test samples in a short time. However, it cannot be determined whether or not endocrine is actually disrupted using the in vitro method because it only determines an activity of binding to a receptor or the like. On the other hand, the actual influence on endocrine is directly examined using the in vivo method in which animals such as rats, frogs, fishes or the like are used. Thus, such a method is necessary in order to determine the influence on a living body or the environment. However, the in vivo method has drawbacks because, for example, its sensitivity is low, it requires complicated operation and, if a number of samples are to be examined, it requires a long time.

For example, a system in which conversion of male into female is monitored to assess an endocrine-disrupting activity has been constructed. The monitoring is carried out by determining the expression of a fish female-specific yolk precursor protein vitellogenin in males using an anti-vitellogenin antibody. However, it is difficult to deal with a number of test samples at a time using the system. The sensitivity of the system in which adult fishes are used for the assessment is supposed to be lower than that of an assessment system in which fries are used because fries are relatively subject to disrupting activities. The system has further problems because it requires a wide space for breeding and a long breeding period. In addition, anti-vitellogenin antibodies specific for the respective fishes to be assessed are required for the system.

An assessment method in which the hatchability of eggs or the number of eggs spawned from an adult fish are used as indexes, and an assessment method in which courtship behavior or the like is monitored have been proposed (Lisa, D. et al., Environmental Toxicology and Chemistry, 17:49-57, 1998). However, it cannot be determined whether or not sexual differentiation is actually disrupted using such methods.

Disruption of sexual differentiation can be examined by determining both the genotypic sex and the phenotypic sex of an individual and comparing them to each other. For example, a system for assaying a disrupting activity in which sex reversal is used as an index has been proposed. In the system, a fry or an egg is bred while administering an environmental hormone thereto, and the phenotypic sex associated with secondary sex characteristics is then determined. For example, in case of medaka, the genotypic sex can be determined by using PCR (Shinoyama, A. et al., The Fish Biology Journal MEDAKA, 10:31-31, 1999), or a medaka strain of which the genotypic sex is linked to pigment expression, d-rR (Yamamoto, T., J. Exp. Zool., 123:571-594, 1958) or Qurt (Wada, H. et al., Zoological Science, 15: 123-126, 1998). It is required to prepare a tissue section to microscopically examine a gonad in order to determine the phenotypic sex of a fry. Thus, it is difficult to deal with a number of test samples. Breeding for at least one month is required in order to determine the phenotypic sex based on the shape of a fin associated with secondary sex characteristics. Furthermore, skill is required for the determination. It is also difficult to deal with a number of test samples using this method. As described above, in fact, the in vivo assay requires a long time from the start of assessment, and it is difficult to assay a number of test samples at a time. However, such an in vivo assay is necessary for assessing chemical substances or water environment, or monitoring water pollution. In addition, construction of a rapid and accurate assay system has been desired.

Kanamori, A. (Molecular Reproduction and Development, 55:31-36, 2000) describes the identification of differences in gene expression between medaka males and females during sex differentiation by subtractive hybridization. Gray, M.A. and Metcalfe, C.D. (Environmental Toxicology and Chemistry, 16:1082-1086, 1997) describe the determination of effects of 4-nonylphenol on the development of primary and secondary sexual characteristics of Japanese medaka.

Examination of an endocrine-disrupting activity may provide an index for assessing the influence of a chemical substance on humans, for determining the influence on living bodies upon its release into the environment or on the ecosystem, or for monitoring water pollution. However, the prior art has drawbacks as described above. Thus, a sensitive and rapid method for determining an endocrine-disrupting activity has been desired.

Summary of Invention

As a result of intensive studies, the present inventors have successfully isolated and identified genes expressed specifically in phenotypic females of medaka during early development for the first time. The present inventors have found that disruption of sexual differentiation can be examined by rapidly determining the phenotypic sex for a fry of medaka using the gene, and comparing the phenotypic sex with the genotypic sex. The present inventors have also found that an endocrine-disrupting activity of a sample can be rapidly determined using the method. The present inventors have successfully constructed a method for rapidly assessing endocrine-disrupting activities of chemical substances, samples of water environment (lakes, marshes, rivers, seas, etc.) or the like in vivo for a number of samples at a time. Thus, the present invention has been completed.

The present invention is outlined as follows.

The first aspect of the present invention relates to a method for assessing a sexual differentiation-disrupting activity of a sample, the method comprising:

(1) administering a sample to be assessed for its sexual differentiation-disrupting activity to a medaka;
(2) determining the genotypic sex of the medaka;
(3) determining the phenotypic sex of the medaka based on the expression of a female-specific gene which has the nucleotide sequence of SEQ ID NO: 5; and
(4) determining if the sexual differentiation is disrupted based on the results of steps (2) and (3) wherein the sexual differentiation is considered to be disrupted if the genotypic sex is different from the phenotypic sex,
wherein the phenotypic sex is determined using a fry of medaka within five days after hatching.

The second aspect of the present invention relates to a method for detecting an endocrine disruptor, comprising assessing a sexual differentiation-disrupting activity by the method of the first aspect.

Detailed Description of the Invention

The present invention is described in detail below.

The present invention may be applied to any samples without limitation, including naturally occurring or artificially synthesized chemical compounds. Such a substance may be subjected to the method of the present invention in an isolated form or in a mixture. The method of the present invention is applicable to a sample from the environment such as river water, soil or the like.

A medaka (Oryzias latipes) is used as an organism according to the method of the present invention. It is widely used as a material for studying genes because it is small and easy to handle, it releases a lot of eggs upon spawning, its generation time is short (about three months), and a genetically homogeneous strain can be established by inbreeding.

A medaka can be bred in distilled water in a 96-well microplate for about one week after hatching. Since feeding is unnecessary, secondary factors such as a disrupting activity due to a bait can be excluded upon assessment.

One can breed a medaka in water containing salt at various concentrations, including fresh water and seawater, at a wide range of temperatures from about 0 to about 30°C. Thus, it can be used for assessment assuming various environments.

There is no specific limitation concerning the medaka to be used according to the present invention. A wild type medaka or a medaka strain of which the genotypic sex is linked to pigment expression such as d-rR or Qurt may be used.

Qurt is a medaka strain heterozygous for the leucophore free (lf) locus, which is closely linked to sex. A female ($X^{1f}/X^{1f}$) individual of Qurt is colorless, whereas a male ($X^{1f}/Y^{+}$) individual is yellow as a result of pigment expression.

The yellow color can be observed for an egg. Therefore, Qurt can be preferably used according to the present invention because its genotypic sex can be readily determined by microscopically examining the egg without extracting the DNA (Zoological Science, 15:123-126, 1998).

There is no specific limitation concerning the method for determining the genotypic sex according to the present invention. For example, the genotypic sex can be determined by genetic analysis of sex chromosomes.

The genotypic sex of medaka is fixed upon fertilization depending on the combination of sex chromosomes as follows: female in case of X/X; and male in case of X/Y. Thus, the genotypic sex can be determined by genetic analysis of sex chromosomes. There is no specific limitation concerning the method for the genetic analysis. For example, the analysis can be carried out by hybridization using a probe that hybridizes with a gene on the sex chromosomes or a PCR using primers that can be used to amplify a gene on the sex chromosomes. If such a method is used to determine the genotypic sex, it is also necessary to prepare both DNA and RNA from an individual. This is because it is necessary to analyze the expression of a female-specific gene using RNA for determining the phenotypic sex as described below. In this case, the head portion of a fry is cut off. The remaining body portion which contains a gonad and the like is used to determine the expression of a gene that is specifically expressed depending on the phenotypic sex as described below. DNA extracted from the head portion is used to determine the genotypic sex. Alternatively, DNA and RNA may be prepared simultaneously using QIAGEN RNA/DNA System (QIAGEN). Furthermore, DNAs can be prepared simultaneously from a number of test samples in a 96-well microplate using DNeasy 96 Tissue Kit (QIAGEN).

If the medaka strain Qurt is used, the difference in pigment expression specific for the genotypic sex can be recognized on the second day after fertilization. Thus, the genotypic sex can be determined for an egg by detecting the pigment without preparing DNA.

A female-specific gene which has the nucleotide sequence of SEQ ID NO:5 is used for determining the phenotypic sex according to the method of the present invention.

The gene may exist on a chromosome with an intron or introns being inserted.

For example, the above-mentioned gene can be isolated as follows.

The sexual differentiation of a medaka is first manifested as a phenomenon that the number of germ cells in a female is about twice as many as that in a male upon hatching, i.e., on about tenth day after fertilization (Satoh, N., Egami, N., J. Embryol. Exp. Morph., 28:385-395, 1972). This is because mitosis is initiated immediately after hatching in a portion of germ cells in a female whereas germ cells in a male do not divide until two months after hatching. It is possible to identify genes expressed in a female-specific manner at an early development stage using the difference in sexual differentiation in germ cell line as an index. Difference in gene expression between a male and a female of medaka can be examined using subtractive hybridization. Genotypic male and female are separated from each other before hatching. RNAs are extracted from the both after hatching. Then, a gene expressed in a female-specific manner can be isolated using subtractive hybridization. If the medaka strain Qurt is used, genotypic male and female can be readily distinguished before hatching on the second day after fertilization using the expression of the pigment gene as an index.

A genomic gene corresponding to each gene can be isolated by screening a genomic library according to a known method using the thus obtained gene as a probe.

The gene can be detected using an oligonucleotide designed based on the nucleotide sequence of the gene. The oligonucleotides for detecting the gene include, but are not limited to, primers that can be used to amplify the gene or a portion thereof according to a gene amplification method, and a probe that is hybridizable with the gene under stringent conditions.

Stringent hybridization conditions include, for example, those as described in T. Maniatis et al. (eds.), Molecular Cloning: A Laboratory Manual 2nd ed., Cold Spring Harbor Laboratory, 1989. Incubation with a probe at 65°C overnight in a solution containing 6 x SSC (1 x SSC: 0.15 M NaCl, 0.015 M sodium citrate, pH 7.0), 0.5% SDS, 5 x Denhardt's and 100 mg/ml herring sperm DNA exemplifies the conditions.

Examples of gene amplification methods that can be used include, but are not limited to, PCR, SDA, NASBA and ICAN (WO 00/56877).

A primer or a probe can be designed at will based on the nucleotide sequence of the gene. Of course, a sequence is selected upon designing such that the primer or the probe does not form a secondary structure within the molecule, and attention is paid such that the melting temperature (Tm value) for the primer or the probe and the corresponding template is set at an appropriate temperature.

The Tm value of a primer or a probe can be determined, for example, according to the following equation:

$$Tm = 81.5 - 16.6(\log_{10}[Na^+]) + 0.41(\%G+C) - (600/N)$$

wherein N is the chain length of the primer or the probe; %G+C is the content of guanine and cytosine residues in the primer or the probe.

If the chain length of the primer or the probe is shorter than 18 bases, the Tm value can be estimated, for example, as the sum of the product of the number of adenine and thymine (A+T) residues multiplied by 2(°C) and the product of the number of G+C residues multiplied by 4(°C), i.e., [(A+T) x 2 + (G+C) x 4].

The chain length of the probe is preferably 15 bases or more, more preferably 18 bases or more in order to avoid nonspecific hybridization.

For example, a primer of 15 to 40 bases in length can be used. In particular, a primer of 17 to 30 bases in length can be preferably used.

Furthermore, it is desirable to design a primer such that the ratio of cytosine (C) and guanine (G) around the 3'-terminus becomes high. A commercially available software for primer designing such as OLIGO™ Primer Analysis software (Takara Shuzo) may be used for designing a primer.

The primer or the probe may have a mutation such as deletion, substitution, insertion or addition of a nucleotide (or nucleotides) in a portion of the sequence as long as it can be used to detect the gene. In case of a primer, it is preferable not to include a mutation or, if any, to minimize mutations around the 3'-terminus of the primer because such a mutation greatly influences the efficiency of primer extension reaction. An appropriate sequence unrelated to the nucleotide sequence of the gene (e.g., a promoter sequence recognized by an RNA polymerase) may be added on the 5' side of the primer.

Optionally, the primer or the probe may be appropriately modified. Addition of a ligand such as biotin or digoxigenin, or a fluorescent substance to a primer or a probe facilitates the detection of the amplification reaction product.

A product of a gene amplification reaction using the primers can be detected by subjecting a portion of the reaction mixture after the amplification reaction to electrophoresis, and then staining the DNAs with ethidium bromide. An amplification product can be detected without electrophoresis by utilizing hybridization. If a modified primer is used, a detection method suitable for the modification can be used.

A kit containing the primer or the probe can be constructed and used for detecting the gene. Such a kit may contain a buffer or an enzyme to be used for an amplification reaction or hybridization. It may further contain a reagent to be used for preparation of a nucleic acid sample from cells or detection of an amplification product in order to make the detection more convenient.

There is no specific limitation concerning the method for detecting the expression of the gene. For example, the expression can be detected by detecting the mRNA transcribed from the gene in RNA prepared from a medaka on the 1st to 5th day after hatching by Northern hybridization, RT-PCR or the like.

RNA can be prepared from a medaka, for example, by directly treating the medaka individual using TRIzol reagent (Gibco-BRL) or the like. Alternatively, RNA and DNA can be simultaneously prepared using QIAGEN RNA/DNA System (QIAGEN). In this case, the DNA can be used for the determination of the genotypic sex. Furthermore, DNAs can be prepared simultaneously from a large number of test samples in a 96-well microplate using RNeasy 96 Kit (QIAGEN).

In order to exclude false positive results due to products amplified from a genomic DNA, it is desirable to use a pair of primers for RT-PCR that can be used to amplify a region of mRNA transcribed from the gene of interest and that is designed such that the corresponding region in the gene contains an intron or introns being inserted. For example, a combination of a primer F1 (SEQ ID NO:24) and a primer R1 (SEQ ID NO:25), a combination of a primer F2 (SEQ ID NO:26) and a primer R2 (SEQ ID NO:27) or the like can be used to detect Gene 5. The primers F1, R1, F2 and R2 have sequences in the exons 2, 8, 2 and 7, respectively.

If the primers F1 and R1 are used, the size of the product amplified from the mRNA is about 730 bp, whereas the size of the product amplified from the genomic DNA is about 1.1 kbp. If the primers F2 and R2 are used, the size of the product amplified from the mRNA is about 590 bp, whereas the size of the product amplified from the genomic DNA is about 0.9 kbp. Thus, the product amplified from the mRNA can be clearly distinguished from the product amplified from the genomic DNA as a background.

More sensitive detection can be accomplished using these primer pairs by carrying out a 1st PCR using the pair of primers F1 and R1 followed by a nested PCR using the pair of primers F2 and R2.

In order to exclude false positive results due to genomic DNA, it is desirable to use a probe for Northern hybridization that hybridizes with a region of mRNA transcribed from the gene of interest and that is designed such that the corresponding region in the gene contains an intron or introns being inserted.

The expression of the gene can be detected using a DNA microarray. A DNA microarray is a material having nucleic acids being immobilized in which a number of different genes or DNA fragments are arrayed and immobilized on a solid phase substrate such as a slide glass. The DNA microarray is used to examine the existence of a nucleic acid in a nucleic acid sample that has a sequence complementary to the DNA immobilized on the microarray by contacting it with a nucleic acid sample (preferably a labeled nucleic acid sample) prepared from a sample for hybridization. Expression of plural genes specifically expressed depending on the phenotypic sex can be monitored at the same time using the microarray.

Also, the expression of the gene can be detected using an antibody to a protein translated from the gene. There is no specific limitation concerning the antibody used as long as it can recognize the protein expressed from the gene. A polyclonal antibody, a monoclonal antibody or the like prepared according to a known method may be used.

A sexual differentiation-disrupting activity of a sample can be assessed by comparing the phenotypic sex with the genotypic sex both determined as described above. An endocrine-disrupting activity of a sample can be assessed according to this method. Although it is not intended to limit the present invention, disruption of sexual differentiation may be represented by expression of a phenotypic female-specific gene in a genotypic male individual, or loss of expression of a phenotypic female-specific gene in a genotypic female individual.

For example, a sexual differentiation-disrupting activity of a sample can be assessed as follows. A medaka egg immediately after fertilization is bred in a test water sample. The genotypic sex and the phenotypic sex are determined for each individual as described above. An individual is determined to be influenced by an sexual differentiation-disrupting activity if the genotypic sex of the individual is different from the phenotypic sex of the same individual. The sexual differentiation-disrupting activity of the sample can be assessed by counting the number of such individuals. If a medaka strain Qurt of which the genotypic sex is linked to pigment expression is used, the relationship between the genotypic sex and the pigment expression may be reversed due to translocation of chromosome with the probability usually at several percentage or less. This problems can be solved by increasing the number of test samples. Translocation of chromosome is rarely observed for a medaka strain d-rR of which the genotypic sex is also linked to pigment expression. Thus, almost no reversion is observed for the relationship between the genotypic sex and the pigment expression if this medaka strain is used.

A kit for assessing a sexual differentiation-disrupting activity using the method of the present invention may contain an oligonucleotide that can be used to detect the gene as described above. Such a kit may contain a buffer or an enzyme to be used for an amplification reaction. It may further contain a reagent to be used for preparation of a nucleic acid sample from cells or detection of an amplification product in order to make the detection convenient.

A kit for detecting an endocrine disruptor using the method of the present invention may contain an oligonucleotide that can be used to detect the gene as described above. Such a kit may contain a buffer or an enzyme to be used for an amplification reaction. It may further contain a reagent to be used for preparation of a nucleic acid sample from cells or detection of an amplification product in order to make the detection convenient.

The phenotypic sex can be determined at an early stage by the fifth day after hatching according to the method of the present invention. Conventionally, the phenotypic sex could be determined only based on the shape change of a fin associated with secondary sex characteristics one month or more after hatching or the like. Furthermore, no special skill is required for the present invention. Thus, it is also possible to deal with a number of test samples according to the method of the present invention. The method is effective in rapidly and conveniently assessing endocrine-disrupting activities of naturally occurring or artificially synthesized chemical substances as well as samples from the environment such as river water or soil.

## Examples

The following examples further illustrate the present invention in detail but are not to be construed to limit the scope thereof.

### Example 1: Identification of female-specific genes by subtractive hybridization

A pair of mature Qurt medakas consisting of one male and one female (Zoological Science, 15:123-126, 1998) was bred in 3 L of green water (purified water containing chlorella from green algae) at 25°C under conditions of a light period for 14 hours and a dark period for 10 hours. They were fed with TetraMin three to five times a day setting the amount of TetraMin such that it was consumed within three to five minutes, and then spawned. Embryos were genotypically classified into males and females by examining the expression of the yellow pigment gene as autofluorescence under a fluorescence microscope on the fourth day after spawning. mRNAs were prepared from samples at four stages (stage 37/39 (2-3 days before hatching), or 1, 5 or 30 day(s) after hatching) according the classification table of Iwamatsu (Iwamatsu, T., Zool. Sci., 11:825-839, 1994). cDNAs were prepared using an oligo(dT) primer and Copy Kit (Invitrogen). The cDNAs were cleaved with a restriction enzyme AluI, ligated to linkers (Wang, Z. and Brown, D., Proc. Natl. Acad. Sci. USA, 88:11505-11509, 1991), and amplified using PCRs. Subtractive hybridization was carried out using cDNAs from a male and a female at each stage. The remaining cDNAs were amplified using PCRs. This process of subtraction/PCR amplification was repeated three times. The amplified cDNAs for the males and the females at the respective stages were cloned into a plasmid to obtain eight cDNA libraries. Fragments inserted in clones selected at random from the respective libraries were isolated and subjected to Southern hybridization to screen for genes expressed in a sex-specific manner at each stage. The starting cDNAs and the cDNAs obtained after three rounds of subtraction/PCR amplification were used as probes for the Southern hybridization. No male-specific positive reaction was observed. Thus, no gene expressed in a male-specific manner could be isolated. Three clones and forty-seven clones were obtained from the 5th and 30th day female libraries, respectively, as clones that exhibit positive reactions in a female-specific manner. The fragments inserted in these clones were used as probes for hybridization with the cDNAs from the 1st, 5th or 30th day male or female (both the starting cDNAs and the cDNAs obtained after three rounds of subtraction/PCR amplification). Based on the results of hybridization, the genes were classified into three groups, i.e., groups of genes expressed in females on the 1st, 5th or 30th day. Nucleotide sequences of two genes classified as those expressed in females on the 1st day (Genes 1 and 2) and nineteen genes classified as those expressed in females on the 5th day (Genes 3-21) were determined. The determined nucleotide sequences of Genes 1-21 are shown as SEQ ID NOS:1-21.

Homology searches of database for the determined gene sequences revealed that the Genes shared homologies with known genes as follows: Gene 1 (SEQ ID NO:1) - gene for FIGα, mouse transcription factor having a basic helix-loop-helix motif; Gene 2 (SEQ ID NO:2) - gene for eIF-4, human cap-binding subunit of elongation initiation factor; Gene 3 (SEQ ID NO:3) - gene encoding rabbit ZPA domain; Gene 4 (SEQ ID NO:4) - gene encoding goldfish ZPB domain; Gene 5 (SEQ ID NO:5) - gene encoding carp ZPC domain; Gene 6 (SEQ ID NO:6) - gene encoding zebra fish ZPC domain; Gene 7 (SEQ ID NO:7) - gene encoding carp ZPC domain; Gene 8 (SEQ ID NO:8) - gene encoding zebra fish ZPC domain; Gene 9 (SEQ ID NO:9) - gene encoding zebra fish ZPC domain; Gene 10 (SEQ ID NO:10) - Xenopus 42Sp43 gene which encodes oocyte-specific RNA storage proteins; Gene 11 (SEQ ID NO:11) - Xenopus 42Sp50 gene; and Gene 12 (SEQ ID NO:12) - rat quinone reductase gene. Genes 13-21 (SEQ ID NOS:13-21) did not share homology with a known gene. It is supposed that they are unknown genes that encode secretory proteins based on the sequence characteristics.

Next, a genomic library was constructed using chromosomal DNA prepared from a medaka according to a known method. Screening was carried out using Gene 1 or 8 as a probe. Corresponding genomic genes were isolated and the nucleotide sequences were determined. Nucleotide sequences of Genomic Gene 22 (corresponding to Gene 1) and Genomic Gene 23 (corresponding to Gene 8) are shown as SEQ ID NOS:22 and 23.

Example 2: Detection of sexual differentiation-disrupting activity of 17 β-estradiol

Pairs of mature Qurt medakas each consisting of one male and one female were bred in 3 L of green water (purified water containing chlorella from green algae) at 25°C under conditions of a light period for 14 hours and a dark period for 10 hours. They were fed with TetraMin three to five times a day setting the amount of TetraMin such that it was consumed within three to five minutes, and then spawned. Eggs which resulted from five pairs were placed in a Petri dish immediately after spawning, separated from each other using tweezers in tap water from which chlorine had been removed and washed. The water was replaced by a 1-ppb 17 β-estradiol (E2) aqueous solution containing 0.1% dimethyl sulfoxide. The mixture was dispensed into wells of a 96-well round bottom microplate (#3797, Corning) which had been extensively washed with ultrapure water such that each well contained one egg. After covering with a lid, the microplate was incubated in an incubator at 25°C. On the third day from the start of incubation, males and females were distinguished by examining the yellow pigment which is expressed in a male-specific manner as autofluorescence using a fluorescence microscope (Nikon). The incubation was further continued, and fries then hatched on the 7th to 9th day after spawning. Fries were collected on the 12th day after spawning (the 3rd to 5th day after hatching), then soaked in RNAlater (#7021, Ambion) and stored at -80°C. RNAs were extracted from the fries using StrataPrep Total RNA Miniprep Kit (#400711, Stratagene). The test samples were soaked in a lysis solution attached to the kit, and homogenized in 1.5-mL tubes using a pellet mixer (Urin Seisakusho). Then, extraction was completed according to the manual attached to the kit. The extracted RNAs were subjected to TaKaRa One Step RNA PCR Kit (AMV) (Takara Shuzo). 1st PCRs were carried out using a pair of primers F1 (SEQ ID NO:24) and R1 (SEQ ID NO:25) which is used to amplify a region of 729 bp in Gene 5 (SEQ ID NO:5), a gene expressed in a female-specific manner. Next, nested PCRs were carried out using a pair of primers F2 (SEQ ID NO:26) and R2 (SEQ ID NO:27) which is used to amplify a region of 593 bp within the 729-bp region using 0.5 μL each of the products of the 1st PCRs as templates. The resulting amplification products were subjected to electrophoresis on 2% agarose gel. The results are shown in Table 1. In the table, the genotypic sex represents the result of microscopic examination, and the phenotypic sex is represented as the result of distinction between a female and a male using the RT-PCR.

Table 1

Control group
(treatment with solvent (water containing 0.1% DMSO))

| Sample No. | 1 | 2 | 3 | 4 | 5 | 6 | 7 | 8 |
|---|---|---|---|---|---|---|---|---|
| Genotypic sex | ♂ | ♂ | ♂ | ♂ | ♀ | ♀ | ♀ | ♀ |
| Expression of Gene 5 | - | - | - | - | + | + | + | + |

(continued)

Treatment with 1 ppb 17 β-estradiol

| Sample No. | 1 | 2 | 3 | 4 | 5 | 6 | 7 | 8 | 9 | 10 |
|---|---|---|---|---|---|---|---|---|---|---|
| Genotypic sex | ♂ | ♂ | ♂ | ♂ | ♂ | ♀ | ♀ | ♀ | ♀ | ♀ |
| Expression of Gene 5 | - | - | - | + | + | - | - | + | + | + |

For the fries hatched from eggs treated with a solvent (water containing 0.1% DMSO) in the control group, the expression of the female-specific gene, Gene 5 was observed only for the genotypic females, and not for the genotypic males. Thus, the genotypic sex was consistent with the phenotypic sex. On the other hand, for the fries hatched from eggs treated with 1 ppb 17 β-estradiol, the expression of Gene 5 was observed for two out of the five genotypic males. Furthermore, the expression of Gene 5 was not observed for two out of the five genotypic females. These results show that the sexual differentiation was disrupted.

Industrial Applicability

The present invention provides a rapid and convenient method for assessing a sexual differentiation-disrupting activity of a sample comprising determining the phenotypic sex using the expression of a gene which has the nucleotide sequence of SEQ ID NO: 5 as an index. The phenotypic sex can be determined within five days after hatching according to the method of the present invention. Conventionally, the phenotypic sex could not be determined until one month after hatching or the like. In addition, determination can be carried out for a number of test samples in a short time according to the method of the preset invention.

A sexual differentiation-disrupting activity of a sample can be determined rapidly and conveniently according to the method of the present invention by administering a sample suspected to have a sexual differentiation-disrupting activity to a medaka, determining the phenotypic sex of the medaka and comparing the result of the determination with the genotypic sex.

Sequence Listing Free Text

SEQ ID NO:1: cDNA for gene 1
SEQ ID NO:2: cDNA for gene 2
SEQ ID NO:3: cDNA for gene 3
SEQ ID NO:4: cDNA for gene 4
SEQ ID NO:5: cDNA for gene 5
SEQ ID NO:6: cDNA for gene 6
SEQ ID NO:7: cDNA for gene 7
SEQ ID NO:8: cDNA for gene 8
SEQ ID NO:9: cDNA for gene 9
SEQ ID NO:10: cDNA for gene 10
SEQ ID NO:11: cDNA for gene 11
SEQ ID NO:12: cDNA for gene 12
SEQ ID NO:13: cDNA for gene 13
SEQ ID NO:14: cDNA for gene 14
SEQ ID NO:15: cDNA for gene 15
SEQ ID NO:16: cDNA for gene 16
SEQ ID NO:17: cDNA for gene 17
SEQ ID NO:18: cDNA for gene 18
SEQ ID NO:19: cDNA for gene 19
SEQ ID NO:20: cDNA for gene 20
SEQ ID NO:21: cDNA for gene 21
SEQ ID NO:22: Genomic DNA for gene 1
SEQ ID NO:23: Genomic DNA for gene 8
SEQ ID NO:24: PCR primer F1
SEQ ID NO:25: PCR primer R1
SEQ ID NO:26: PCR primer F2

SEQ ID NO:27: PCR primer R2

## Claims

1. A method for assessing a sexual differentiation-disrupting activity of a sample, the method comprising:

    (1) administering a sample to be assessed for its sexual differentiation-disrupting activity to a medaka;
    (2) determining the genotypic sex of the medaka;
    (3) determining the phenotypic sex of the medaka based on the expression of a female-specific gene which has the nucleotide sequence of SEQ ID NO: 5; and
    (4) determining if the sexual differentiation is disrupted based on the results of steps (2) and (3), wherein the sexual differentiation is considered to be disrupted if the genotypic sex is different from the phenotypic sex,

    wherein the phenotypic sex is determined using a fry of medaka within five days after hatching.

2. The method according to claim 1, wherein the genotypic sex is determined using an egg of medaka before hatching or a fry of medaka within five days after hatching.

3. The method according to claim 1 or 2, wherein the sample is administered to an egg of medaka before hatching or a fry of medaka within five days after hatching.

4. The method according to any one of claims 1 to 3, wherein a medaka strain of which the genotypic sex is linked to pigment expression is used.

5. The method according to any one of claims 1 to 4, wherein the expression of the gene is examined using the generation of the mRNA for the gene as an index.

6. A method for detecting an endocrine disruptor, comprising assessing a sexual differentiation-disrupting activity by the method defined by any one of claims 1 to 5.

## Patentansprüche

1. Verfahren zur Bewertung einer die geschlechtliche Differenzierung störenden Aktivität einer Probe, wobei das Verfahren umfasst:

    (1) Verabreichen einer Probe, die bezüglich ihrer die geschlechtliche Differenzierung störenden Aktivität bewertet werden soll, an einen Medaka,
    (2) Bestimmen des genotypischen Geschlechts des Medaka,
    (3) Bestimmen des phänotypischen Geschlechts des Medaka auf der Basis der Expression eines für Weibchen spezifischen Gens, das die Nukleotidsequenz von SEQ ID NO: 5 aufweist, und
    (4) Bestimmen, ob die geschlechtliche Differenzierung gestört wird, auf der Basis der Ergebnisse der Schritte (2) und (3), wobei die geschlechtliche Differenzierung als gestört betrachtet wird, falls das genotypische Geschlecht sich von dem phänotypischen Geschlecht unterscheidet,

    wobei das phänotypische Geschlecht unter Verwendung einer Fischbrut des Medaka innerhalb von fünf Tagen nach Schlüpfen bestimmt wird.

2. Verfahren gemäß Anspruch 1, wobei das genotypische Geschlecht unter Verwendung eines Eis eines Medaka vor Schlüpfen oder einer Fischbrut eines Medaka innerhalb von fünf Tagen nach Schlüpfen bestimmt wird.

3. Verfahren gemäß Anspruch 1 oder 2, wobei die Probe an ein Ei eines Medaka vor Schlüpfen oder an eine Fischbrut eines Medaka innerhalb von fünf Tagen nach Schlüpfen verabreicht wird.

4. Verfahren gemäß einem der Ansprüche 1 bis 3, wobei ein Stamm eines Medaka verwendet wird, bei dem das genotypische Geschlecht mit einer Pigmentexpression gekoppelt ist.

5. Verfahren gemäß einem der Ansprüche 1 bis 4, wobei die Expression des Gens unter Zuhilfenahme der Bildung der mRNA des Gens als Index untersucht wird.

**6.** Verfahren zum Nachweis eines endokrinen Störfaktors, umfassend ein Bewerten einer die geschlechtliche Differenzierung störenden Aktivität durch das in einem der Ansprüche 1 bis 5 definierte Verfahren.

## Revendications

**1.** Méthode destinée à évaluer une activité perturbatrice de la différenciation sexuelle d'un échantillon, la méthode comprenant :

(1) l'administration d'un échantillon à évaluer en ce qui concerne son activité perturbatrice de la différenciation sexuelle à un medaka *(Oryzia latipes) ;*
(2) la détermination du sexe génotypique du medaka ;
(3) la détermination du sexe phénotypique du medaka basée sur l'expression d'un gène femelle spécifique qui présente la séquence nucléotidique de SEQ ID NO :5 ; et
(4) le fait de déterminer si la différenciation sexuelle est perturbée sur la base des résultats des étapes (2) et (3), dans laquelle la différenciation sexuelle est considérée comme perturbée si le sexe génotypique est différent du sexe phénotypique,

dans laquelle le sexe phénotypique est déterminé en utilisant un alevin de medaka dans les cinq jours suivant l'éclosion.

**2.** Méthode de la revendication 1, dans laquelle le sexe génotypique est déterminé en utilisant un oeuf de medaka avant éclosion ou un alevin de medaka dans les cinq jours suivant l'éclosion.

**3.** Méthode selon la revendication 1 ou 2, dans laquelle l'échantillon est administré à un oeuf de medaka avant éclosion ou à un alevin de medaka dans les cinq jours suivant l'éclosion.

**4.** Méthode selon l'une quelconque des revendications 1 à 3, dans laquelle une souche de medaka dont le sexe génotypique est lié à l'expression d'un pigment est utilisée.

**5.** Méthode selon l'une quelconque des revendications 1 à 4, dans laquelle l'expression du gène est examinée en utilisant la production de l'ARNm du gène comme indice.

**6.** Méthode pour détecter un perturbateur endocrinien, comprenant l'évaluation d'une activité perturbatrice de la différenciation sexuelle par la méthode définie par l'une quelconque des revendications 1 à 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0056877 A
- EP 00985852 A
- JP 11372602 A

### Non-patent literature cited in the description


- *Analytical Chemistry,* 1998, vol. 70 (15), 528A-532A
- **LISA, D. et al.** *Environmental Toxicology and Chemistry,* 1998, vol. 17, 49-57
- **SHINOYAMA, A. et al.** *The Fish Biology Journal MEDAKA,* 1999, vol. 10, 31-31
- **YAMAMOTO, T.** *J. Exp. Zool.,* 1958, vol. 123, 571-594
- **WADA, H. et al.** *Zoological Science,* 1998, vol. 15, 123-126
- **KANAMORI, A.** *Molecular Reproduction and Development,* 2000, vol. 55, 31-36
- **GRAY, M.A. ; METCALFE, C.D.** *Environmental Toxicology and Chemistry,* 1997, vol. 16, 1082-1086
- *Zoological Science,* 1998, vol. 15, 123-126
- **SATOH, N. ; EGAMI, N.** *J. Embryol. Exp. Morph.,* 1972, vol. 28, 385-395
- Molecular Cloning: A Laboratory Manual. Cold Spring Harbor Laboratory, 1989
- **IWAMATSU, T.** *Zool. Sci.,* 1994, vol. 11, 825-839
- **WANG, Z. ; BROWN, D.** *Proc. Natl. Acad. Sci. USA,* 1991, vol. 88, 11505-11509